# EUROPÄISCHE PATENTSCHRIFT

(11) **EP 2 712 630 B1**
(45) Veröffentlichungstag und Bekanntmachung des Hinweises auf die Patenterteilung: **09.12.2015**
(21) Anmeldenummer: 12006716.0
(22) Anmeldetag: 26.09.2012
(51) Int. Cl.: C12N 15/85

(54) **Mitochondrialer Expressionsvektor und Verfahren zur Transformation von Mitochondrien**
Mitochondrial expression vector and process for the transformation of mitochondria
Vecteur d'expression mitochondrial et procédé de transformation de mitochondries

(43) Veröffentlichungstag der Anmeldung: 02.04.2014
(73) Patentinhaber: Universität Leipzig, 04109 Leipzig (DE)
(72) Erfinder: Seibel, Peter, 97292 Uettingen (DE)
(74) Vertreter: Schüssler, Andrea

(56) Entgegenhaltungen:
- WO-A2-2006/117250
- WO-A2-2010/003540
- US-B2- 7 279 326
- JOHNSON S A ET AL: "MITOCHONDRIAL TRANSFORMATION IN YEAST BY BOMBARDMENT WITH MICROPROJECTILES", SCIENCE (WASHINGTON D C), Bd. 240, Nr. 4858, 1988, Seiten 1538-1541, XP002693220, ISSN: 0036-8075

### SEQUENCE LISTING

<110> Universität Leipzig
<120> Verfahren zur Transformation von Mitochondrien
<160> 8
<170> BiSSAP 1.0
<210> 1
   <211> 29
   <212> DNA
   <213> Artificial Sequence
<220>
   <221> source
   <222> 1..29
   <223> /mol_type="DNA" /note="EGFP-001-FOR" /organism="Artificial Sequence"
<400> 1
   cttttggtct caatgatggt gagcaaggg 29
<210> 2
   <211> 30
   <212> DNA
   <213> Artificial Sequence
<220>
   <221> source
   <222> 1..30
   <223> /mol_type="DNA" /note="EGFP-716-REV" /organism="Artificial Sequence"
<400> 2
   gttagtggtc tctatttgta cagctcgtcc 30
<210> 3
   <211> 36
   <212> DNA
   <213> Artificial Sequence
<220>
   <221> source
   <222> 1..36
   <223> /mol_type="DNA" /note="EGFP-156-FOR" /organism="Artificial Sequence"
<400> 3
   caagctgccc gtgccctgac ccaccctcgt gaccac 36
<210> 4
   <211> 36
   <212> DNA
   <213> Artificial Sequence
<220>
   <221> source
   <222> 1..36
   <223> /mol_type="DNA" /note="EGFP-191-REV" /organism="Artificial Sequence"
<400> 4
   gtggtcacga gggtgggtca gggcacgggc agcttg 36
<210> 5
   <211> 38
   <212> DNA
   <213> Artificial Sequence
<220>
   <221> source
   <222> 1..38
   <223> /mol_type="DNA" /note="01562-FOR" /organism="Artificial Sequence"
<400> 5
   gcgctgcagt aacatggtaa gtgtactgga aagtgcac 38
<210> 6
   <211> 29
   <212> DNA
   <213> Artificial Sequence
<220>
   <221> source
   <222> 1..29
   <223> /mol_type="DNA" /note="03351-REV" /organism="Artificial Sequence"
<400> 6
   aatctcgaga ttagaatggg tacaatgag 29
<210> 7
   <211> 720
   <212> RNA
   <213> Artificial Sequence
<220>
   <221> source
   <222> 1..720
   <223> /mol_type="RNA" /note="mto-GFP RNA" /organism="Artificial Sequence"
<400> 7
   augguaucca aaggcgaaga acuauucacc ggcguaguac ccauccuagu agaacuagac    60
   ggcgacguaa acggacacaa auucuccgua uccggcgaag gcgaaggcga cgccaccuac   120
   ggcaaacuaa cccuaaaauu cauuugcacc accggcaaac uacccguacc cugacccacc   180
   cuaguaacca cccuaaccua cggcguacaa ugcuucuccc gauaccccga ccacauaaaa   240
   caacacgacu ucuucaaauc cgccauaccc gaaggcuaug uccaagaacg aaccaucuuc   300
   uucaaagacg acggcaacua caaaacacga gccgaaguaa aauucgaggg cgacacccua   360
   guaaaccgaa ucgaacuaaa aggcaucgac uucaaagagg acggcaacau ccuaggccac   420
   aaacuagaau acaacuacaa cucccacaac gucuauauca uagccgacaa acaaaaaaac   480
   ggcaucaaag uaaacuucaa aauccgacac aacaucgagg acggcuccgu acaacuagcc   540
   gaccacuacc aacaaaacac ccccaucggc gacggccccg uacuacuacc cgacaaccac   600
   uaccuaucca cccaauccgc ccuauccaaa gaccccaacg aaaaacgcga ccacauaguc   660
   cuacuagagu ucguaaccgc cgcaggcauc acccuaggca uagacgaacu auacaaauaa   720
<210> 8
   <211> 239
   <212> PRT
   <213> Artificial Sequence
<220>
   <221> SOURCE
   <222> 1..239
   <223> /mol_type="protein" /note="mtoGFP AS" /organism="Artificial Sequence"
<400> 8
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235

## Beschreibung

Die vorliegende Erfindung betrifft ein Verfahren zum Einbringen exogener DNA in Mitochondrien von Säugerzellen.

### Einleitung

Mitochondriale Erkrankungen bzw. Mitochondriopathien führen zum Versagen des zellulären Energiestoffwechsels und spielen heute eine wichtige Rolle in der pädiatrischen und neurologischen Klinik, wobei die Prävalenz dieser Erkrankungen immer noch schwer abzuschätzen ist (ca. 1:5.000; Friedrich-Baur-Institut).

Sehr häufig treten bei Mitochondriopathien typische Symptomenkomplexe auf, zu denen:
- Muskelschwächen
- verschlechterte Sehkraft bis hin zur Erblindung
- epileptische Anfälle und
- Gleichgewichtsstörungen
gezählt werden. Aufgrund ihrer Schlüsselstellung im aeroben Stoffwechsel der Zelle führt eine Störung der Mitochondrienfunktion allerdings meist zu einem klinischen Mischbild mit Multiorganbeteiligung, bei dem besonders die Gewebe mit hohem Energiebedarf betroffen sind. Das sind das zentrale Nervensystem (Epilepsie, Ataxie, Leukodystrophie, Leigh-Syndrom, mentale Retardierung), die Muskulatur (Myopathie, Muskelschmerzen, Kardiomyopathie, externe Ophthalmoplegie), die endokrinen Organe (Hypothyreose, Hypoparathryreoidismus, Diabetes mellitus Typ I), die Leber (akutes Leberversagen, Leberzirrhose), die Sinnesorgane (Nervus-opticus-Atrophie, Retinitis pigmentosa, Innenohrschwerhörigkeit), das Knochenmark (aregeneratorische Anämie) und die Nieren (Niereninsuffizienz, Fanconi-Syndrom).

Im Folgenden sind die wichtigsten Mitochondriopathien aufgeführt, die durch Akronyme anhand der Symptome bezeichnet werden. MELAS: "Mitochondriale Enzephalopathie, Laktatazidose und schlaganfallähnliche Episoden", MERRF: "Myoklonusepilepsie mit Ragged-Red-Fibers", NARP: "Neuropathie, Ataxie, Retinitis pigmentosa", CPEO: "chronisch progressive externe Ophthalmoplegie (Lähmung der äußeren Augenmuskeln), KSS: "Kearns-Sayre-Syndrom" (eine spezielle Variante der CPEO, multisystemische Erkrankung) und LHON: "Leber'sche hereditäre Optikusneuropathie" (Leitlinien Pädiatrie, A13 Mitochondriale Erkrankungen, Schuelke, M., Stand Dezember 2010, Elsevier GmbH, München; Finsterer, J., Eur. J. Paediatr. Neurol., 2010, 14: 29-44).

Ursachen können genetisch bedingte Defekte in der Pyruvatdehydrogenase, der Pyruvatcarboxylase, in den Proteinen des Zitratzyklus, in den Atmungskettenkomplexen I bis IV oder den ATP-Transportproteinen sein, wobei weitere genetische Defekte Proteine oder Proteinkomplexe betreffen können, welche an der Regulation der mitochondrialen Replikation, Transkription oder der Translation beteiligt sind.

Da die oben genannten Proteine sowohl durch die mitochondriale DNA (mtDNA; maternaler Erbgang) als auch durch das Kerngenom (nDNA; Mendel'scher Erbgang) kodiert werden, können Mutationen in beiden Genomen die Auslöser dieser Erkrankungen darstellen. Allerdings lassen sich Veränderungen der mitochondrialen DNA - im Gegensatz zum Kerngenom - bisher nicht korrigieren, weshalb bei einem großen Teil der mitochondrialen Erkrankungen nur die Symptome und nicht die Auslöser behandelt werden können. Beispielsweise werden mitochondriale Erkrankungen mit der Gabe stoffwechselaktiver Substanzen (z.B. Cofaktoren von Atmungskettenenzymen) therapiert, die die Defekte im oxidativen Stoffwechsel kompensieren sollen. Allerdings konnte diese Therapieform nur in Ausnahmefällen eine deutliche Verbesserung des Gesundheitszustands des Patienten erzielen, wobei eine Heilung ausgehend von Therapieansätzen, die auf der Behandlung von Symptomen basieren, nicht zu erwarten ist (Chinnery, P. F and D. M. Turnbull, Am. J. Med. Genet., 2001, 106: 94-101). Somit sind Therapien, die auf den Krankheitsauslöser der Erkrankung abzielen, als chancenreicher anzusehen.

Die somatische Gentherapie stellt eine mögliche Therapieform mitochondrialer Erkrankungen dar, bei der der Gendefekt der mitochondrialen DNA korrigiert wird. Dazu müsste der Anteil der mutierten mtDNA unter den für die Ausprägung klinischer Symptome notwendigen Schwellenwert gedrückt werden, um die Wiederherstellung des normalen Phänotyps zu ermöglichen. Ideal wäre jedoch eine vollständige Entfernung der mutierten DNA.

Eine somatische Gentherapie kann mittels unterschiedlicher Strategien erfolgen:
- Kompetitierung der mutierten Gene durch Einschleusen intakter replikativer und transkriptionsaktiver Gene;
- Austausch der mutierten Gene gegen intakte Gene durch Rekombination;
- Inhibierung der Replikation mutierter mtDNA durch Antisense-Oligonukleotide;
- Degradation mutierter mtDNA.

Allerdings sind die ersten drei Strategien auf den Transport von Nukleinsäuren in die Mitochondrien angewiesen. Bislang stellt dies jedoch eine große Hürde dar. Der aktive Transport von kernkodierten RNAs und tRNAs in die Mitochondrien ist bei Hefen (Martin, R. P. et al., Biochemistry, 1979, 18: 4600-4605), Protozoen (Simpson L. and J. Shaw, Cell, 1989, 57: 355-366) und Pflanzen (Gray, M. W. and Boer, P. H., Philos. Trans. R. Soc. London B Biol. Sci., 1988, 319: 135-147) bekannt. An isolierten humanen Mitochondrien wurde ebenfalls der Import einer Hefe-tRNA dokumentiert (Entelis, N. S. et al., J. Biol. Chem., 2001, 276: 45642-45653). Allerdings ist es bisher nicht gelungen, den Import größerer Nukleinsäuren in Säuger-Mitochondrien *in-vivo* nachzuweisen.

Eine Möglichkeit besteht in der Nutzung physikalischer Transformationsmethoden. So wurde bereits DNA durch Elektroporation in Mitochondrien eingeführt, jedoch waren diese Mitochondrien vorher aus den Zellen isoliert worden (Collombet, J. M. et al., J. Biol. Chem., 1997, 272: 5342-5347). Als weitere physikalische Methode wurde die *Gene Gun* bzw. *Particle Gun* eingesetzt, bei der Mikroprojektile aus Gold oder Wolfram genutzt werden, um DNA in Zellen zu bringen. Die DNA wird dabei an die Oberfläche der Partikel gebunden und kann am Zielort wieder freigesetzt werden. Bei der Transformation von Chloroplasten in Pflanzen wird diese Methode schon erfolgreich eingesetzt (Kanevski, I. and P. Maliga, Proc. Natl. Acad. Sci., 1994, 91: 1969-1973; Barone, P. et al., J. Exp. Bot., 2009, 60: 3195-3202; De Marchis, F. et al., Transgenic Res., 2009, 18: 17-30). Auch in der Grünalge *Chlamydomonas reinhardtii* gelang die Transformation von Chloroplasten und Mitochondrien (Boynton, J. E. et al., Science, 1988, 240: 1534-1538; Yamasaki, T. et al., Plant Mol. Biol., 2005, 58: 515-527; Remacle, C. et al., Proc. Natl. Acad. Sci. U.S.A., 2006, 103: 4771-4776). In Hefezellen konnten ebenfalls Mitochondrien mittels der *Gene Gun* mit exogener DNA beladen werden (Fox, T. D. et al., Proc. Natl. Acad. Sci. U.S.A., 1988, 85: 7288-7292; Johnston, S. A. et al., Science, 1988, 240: 1538-1541; Sulo, P. et al., Nucleic Acid Res., 1995, 23: 856-860; Meunier, B., Biochem. J., 2001, 354: 407-412).

Diese positiven Ergebnisse beruhen darauf, dass die Zielorganellen in den verwendeten Organismen im Vergleich zu Mitochondrien in Säugerzellen sehr groß sind. Da die verwendeten Partikel einen Durchmesser von mindestens 0,3 µm haben, ist es allerdings nahezu unmöglich, die kleinen Mitochondrien von Säugerzellen zu penetrieren, ohne sie dabei zu zerstören. Aufgrund dieses Problems ist auch eine weitere physikalische Transformations-Methode, die Mikroinjektion, in Mitochondrien bisher nicht realisierbar.

In der Patentanmeldung PCT/EP2009/004454 wird ein *in-vitro*-Verfahren zur reversiblen Induktion von Megamitochondrien beschrieben, bei dem die Säugerzellen in einem Kulturmedium wachsen, das mit Milchsäure auf einen pH-Wert zwischen 5,6 und 6,7 angesäuert worden war, was zur Ausbildung von Megamitochondrien führte. Diese Megamitochondrien sind im Vergleich zu nicht induzierten Mitochondrien größer, so dass postuliert wird, dass sie penetriert werden können, ohne sie dabei zu zerstören. Allerdings ist die Beschreibung der Patentanmeldung PCT/EP2009/004454 auf das Verfahren zur reversiblen Induktion von Megamitochondrien beschränkt, wobei ihre Transfektion und ihr Einsatz zur Untersuchung bzw. Behandlung von Mitochondriopathien lediglich postuliert werden. Zudem führt die Induktion mittels Milchsäure lediglich zur Bildung von Megamitochondrien mit Durchmessern von maximal 2-3 µm führen, die kleiner als die durchschnittliche Größe eines Zellkerns sind, wodurch keine idealen Voraussetzungen zur Transfektion mittels einer physikalischen Transfektionsmethode, z.B. Mikroinjektion, bereit gestellt werden.

### Problem der Erfindung

Somit liegt das Problem der vorliegenden Erfindung in der Bereitstellung eines Verfahrens zum Einbringen exogener DNA in Mitochondrien von Säugerzellen.

### Zusammenfassung der Erfindung

Das technische Problem der Erfindung wird durch den Gegenstand der unabhängigen Ansprüche 1, 11 und 12 gelöst, wobei bevorzugte Ausführungsformen Gegenstand der abhängigen Ansprüche sind.

Die Lösung des oben genannten technischen Problems liegt insbesondere in der Bereitstellung physikalischer Transfektionsmethoden, mittels derer exogene DNA, die in einem mitochondrialen Expressionsvektor integriert ist, in die Mitochondrien von Säugerzellen *in vivo* oder *in vitro* eingebracht wird, wobei die Mitochondrien vor Anwendung der physikalischen Transfektionsmethode zu Megamitochondrien induziert werden.

Im Rahmen der vorliegenden Erfindung werden mitochondriale Expressionsvektoren beschrieben, die dazu verwendet werden können, exogene DNA aufzunehmen und die dazu in der Lage sind, die exogene DNA ausschließlich in Mitochondrien zu exprimieren.

Der Gegenstand der vorliegenden Erfindung ist auf ein verbessertes Verfahren zur reversiblen Induktion von Megamitochondrien gerichtet, da die reversible Induktion der vorliegenden Erfindung zu Megamitochondrien mit Durchmessern führt, die größer als die durchschnittliche Größe eines Zellkerns sind, d.h. eine Größe von bis zu 10 µm aufweisen. Somit stellt die vorliegende Erfindung ideale Voraussetzungen zur Transfektion mittels einer physikalischen Transfektionsmethode, z.B. Mikroinjektion, bereit.

Darüber hinaus werden physikalische Transfektionsmethoden offenbart, mittels derer Megamitochondrien von Säugerzellen transfiziert werden können.

Außerdem stellt die vorliegende Erfindung ein Verfahren zum Einbringen exogener DNA in Mitochondrien von Säugerzellen bereit, das die folgenden Schritte umfasst:
(i) Konstruktion eines mitochondrialen Expressionsvektors,
(ii) reversible Induktion von Megamitochondrien und
(iii) Transfektion der Megamitochondrien mit dem mitochondrialen Expressionsvektor mittels einer physikalischen Transfektionsmethode.

Der mitochondriale Expressionsvektor muss, um seine Funktion erfüllen zu können, mindestens zwei Bestandteile aufweisen: exogene DNA, d.h. ein zu exprimierendes Gen und/oder ein Selektionsmarker, und einen mitochondrialen Promotorbereich. In einer bevorzugten Ausführungsform sind noch andere Bestandteile vorhanden, wie beispielsweise ein Selektionsmarker bzw. Reportergene, Signalsequenzen bzw. Transkriptions-Terminations-Sequenzen (TSS), Replikationsursprünge und/oder ein Sicherheitsmechanismus.

Vor dem Einbringen dieser mitochondrialen Expressionsvektoren in die Mitochondrien ist es notwendig, die Mitochondrien reversibel zu Megamitochondrien zu induzieren. Die reversible Induktion der Megamitochondrien erfolgt vorzugsweise durch Ansäuerung bzw. Azidifizierung des Kulturmediums, das die Säugerzellen umfasst. Die Ansäuerung des Kulturmediums erfolgt entweder durch Zugabe oder durch Entstehenlassen von pH-Wert senkenden Substanzen, vorzugsweise von Säuren oder Salzen davon, bevorzugt Milchsäure, Essigsäure oder Natriumacetat, vorzugsweise Essigsäure oder Natriumacetat, wobei der pH-Wert im Kulturmedium vorzugsweise auf zwischen 5,3 und 6,7 eingestellt wird, um ein saures Kulturmedium zu erzeugen. Die Konzentrationen der Säuren oder der Salze davon liegen im angesäuerten Kulturmedium vorzugsweise bei zwischen 5 bis 100 mM, wobei die bevorzugte Konzentration für Essigsäure bei zwischen 30 bis 35 mM und für Natriumacetat bei zwischen 50 bis 60 mM liegt. Die Säugerzellen werden zur reversiblen Induktion der Megamitochondrien gemäß der vorliegenden Erfindung im angesäuerten Medium vorzugsweise für zwischen 20 min und 2 Tagen inkubiert, wobei die Induktion der Megamitochondrien durch Austauschen des angesäuerten Kulturmediums mit einem Kulturmedium, das einen pH-Wert ≥ 7,0, vorzugsweise einen pH-Wert ≥ 7,4 und keine zusätzlichen pH-Wert senkenden Substanzen aufweist, reversibel ist.

Die reversible Induktion der Megamitochondrien bzw. die Auswahl der/des für die Ansäuerung verwendeten Säure/Salzes erfolgt in Abhängigkeit zur physikalischen Transfektionsmethode, wobei die physikalische Transfektionsmethode aus der Gruppe ausgewählt ist, die aus dem Beschießen der Zellen mit DNA-beschichteten Mikropartikeln in einer Genkanone, der Transfektion mithilfe magnetischer Partikel und der Mikroinjektion besteht, wobei die Mikroinjektion die bevorzugteste Methode darstellt, da bei der Mikroinjektion sehr große Mitochondrien (vorzugsweise > 3µm) verwendet werden und man somit optisch verfolgen kann, ob die Mitochondrien wirklich transfiziert bzw. penetriert wurden. In einer spezifischen Ausführungsform der vorliegenden Erfindung werden vor dem Beschießen der Säugerzellen mit DNA-beschichteten Goldpartikeln in einer Genkanone oder vor der Transfektion mithilfe magnetischer Partikel die Megamitochondrien durch Ansäuerung des Kulturmediums mittels Milchsäure und vor der Mikroinjektion der Säugerzellen die Megamitochondrien durch Ansäuerung des Kulturmediums mittels Zugabe von Natriumacetat oder Essigsäure induziert.

### Kurze Beschreibung der Abbildungen

Abbildung 1 zeigt die Vektorkarte von pMAG11-1.
Abbildung 2A zeigt die Vektorkarte von pEGFP-Mito.
Abbildung 2B zeigt die Vektorkarte von pEGFP-OMP, der das Gen für EGFP trägt, an dessen C-Terminus das Signalpeptid der menschlichen Cytochrom-c-Oxidase-Untereinheit 8 fusioniert wurde. Dadurch wird das Protein in die Matrix der Mitochondrien transportiert.
Abbildung 2C zeigt die Vektorkarte von pEGFP-N1. Der Vektor pEGFP-N1 der Firma Clontech ermöglicht die Expression eines Fusionsproteins, an dessen N-Terminus EGFP lokalisiert ist. Wird der Vektor ohne einklonierte Sequenzen eingesetzt, ist das EGFP im gesamten Cytoplasma und dem Zellkern sichtbar.
Abbildung 3 zeigt die Vektorkarte von pMAG2-2.
Abbildung 4 zeigt die Vektorkarte von pMAG11-2.
Abbildung 5 zeigt die Vektorkarte von pMAG2-1.
Abbildung 6 zeigt die Vektorkarte von pMAG12-1.
Abbildung 7 zeigt die Vektorkarte von pMAG13-1.
Abbildung 8 zeigt die Vektorkarte von pMAG14-1.
Abbildung 9 zeigt die Vektorkarte von pCRII-TOPO-mtEGFP.
Abbildung 10 zeigt die Vektorkarte von pMAG14-2.
Abbildung 11 zeigt die Vektorkarte von pMAG14-3.
Abbildung 12 zeigt die Induktion von Megamitochondrien durch Natriumacetat, d.h. Fluoreszenz- und Phasenkontrastaufnahmen von mit Natriumacetat induzierten Megmitochondrien in der mit pEGFP-Mito transfizierten Zelllinie 143.B.TK⁻. Die Eichstriche entsprechen 10 µm.
Abbildung 13 zeigt Megamitochondrien. Aufnahmen der mit pEGFP-OMP stabil transfizierten Zelllinie 143B.TK⁻. Megamitochondrien sind mit Pfeilen markiert. A+D: Fluoreszenzaufnahmen, B+E: Phasenkontrastaufnahmen, C+F: Überlagerung von A und B bzw. D und E. Die Eichstriche entsprechen 10 µm.
Abbildung 14 zeigt die Induktion von Megamitochondrien durch Essigsäure, d.h. Fluoreszenz- und Phasenkontrastaufnahmen von mit Essigsäure induzierten Megamitochondrien in der mit pEGFP-Mito transfizierten Zelllinie 143B.TK-. Die Eichstriche entsprechen 10 µm.
Abbildung 15 zeigt die Optimierung der Versuchsparameter zur Nutzung der Genkanone, 15 und 20 in Hg. A-D bzw. M-P: Fluoreszenzaufnahmen der Zelllinie 143B.TK-; E-H bzw. Q-T: Phasenkontrastaufnahmen; I-L bzw. U-X: Überlagerung aus Fluoreszenz- und Phasenkontrastaufnahmen. Die Eichstriche entsprechen 100 µm.
Abbildung 16 zeigt die Optimierung der Versuchsparameter zur Nutzung der Genkanone, 25 und 27 in Hg. A-D bzw. M-P: Fluoreszenzaufnahmen der Zelllinie 143B.TK-; E-H bzw. Q-T: Phasenkontrastaufnahmen; I-L bzw. U-X: Überlagerung aus Fluoreszenz- und Phasenkontrastaufnahmen. Die Eichstriche entsprechen 100 µm.
Abbildung 17 zeigt die Vergleichs-Transfektionsrate von FuGENE^{®} HD. Die Eichstriche entsprechen 100 µm.
Abbildung 18 zeigt die Induktion von Megamitochondrien durch Milchsäuremedium bzw. Zugabe von Valinomycin. Fluoreszenzaufnahmen, A-D: 143B.TK⁻ +pEGFP-Mito i n normalem Kulturmedium, E-H: 143B.TK⁻+pEGFP-Mito in mit Milchsäure auf einen pH-Wert von 6,3 eingestelltem Kulturmedium, I-L: 143B.TK⁻+pEGFP-Mito in Kulturmedium mit 10 µM Valinomycin. Die Eichstriche entsprechen 100 µm.
Abbildung 19 zeigt die Wachstumshemmung durch Behandlung mit Valinomycin. Phasenkontrastaufnahmen, A-C: 143B.TK⁻ ohne Valinomycinbehandlung, D-F: 143B.TK⁻ nach einstündiger Valinomycinbehandlung, G-I: 143B.TK⁻ nach zweistündiger Valinomycinbehandlung, J-L: 143B.TK⁻ nach vierstündiger Valinomycinbehandlung. Die Eichstriche entsprechen 100 µm.
Abbildung 20 zeigt die Sichtbarkeit der Megamitochondrien im Phasenkontrast. Phasenkontrastaufnahmen von 143B.TK⁻ in mit Milchsäure auf einen pH-Wert von 6,3 eingestelltem Kulturmedium. Die Eichstriche entsprechen 10 µm.
Abbildung 21 zeigt die Aggregation der Goldpartikel durch mtDNA. Phasenkontrastaufnahmen von Goldpartikeln nach dem Beschuss einer Kulturschale. A: mit pMAG13-1 beschichtete Goldpartikel als Kontrolle, B: mit mtDNA beschichtete Goldpartikel, C: Vergrößerung von B. Die Eichstriche entsprechen 30 µm.
Abbildung 22 zeigt fluoreszierende Mitochondrien nach Genkanonen-Beschuss mit pMAG13-1. A: Fluoreszenzaufnahme (verstärkt) der Zellen, B: Phasenkontrastaufnahme der Zellen, C: Überlagerung von A und B. Die Eichstriche entsprechen 20 µm.
Abbildung 23 zeigt die Vektorkarte von pMAG17-1.
Abbildung 24 zeigt die Fluoreszenz der MATra-A-Partikel. Aufnahmen von MATra-A-Partikeln ohne DNA auf 143B.TK⁻-Zellen, A: Fluoreszenzaufnahme B: Phasenkontrastaufnahme, C: Überlagerung von A und B. Die Eichstriche entsprechen 20 µm.
Abbildung 25 zeigt fluoreszierende Mitochondrien nach Mikroinjektion mit pMAG13-1. A: Fluoreszenzaufnahme (verstärkt) der Zellen, B: Phasenkontrastaufnahme der Zellen, C: Überlagerung von A und B. Die Eichstriche entsprechen 20 µm.
Abbildung 26 zeigt die optimierte Sequenz mtoGFP, wobei die obere Zeile jeweils die RNA-Sequenz (U statt T) angibt und die untere Zeile die daraus resultierende Aminosäuresequenz nach einer Translation in den Mitochondrien aufführt (* = Stop). Das veränderte Tryptophan-Codon Trp58 fettgedruckt und unterstrichen.

### Ausführliche Beschreibung der Erfindung

Die vorliegende Erfindung stellt physikalische Transfektionsmethoden bereit, mittels derer exogene DNA, die in einem mitochondrialen Expressionsvektor integriert ist, in die Mitochondrien von Säugerzellen bzw. Zellen *in vivo* oder *in vitro* eingebracht wird, wobei die Mitochondrien vor Anwendung der physikalischen Transfektionsmethode zu Megamitochondrien induziert werden. Diese Transfektionsmethoden können beispielsweise dazu verwendet werden, mitochondriale Erkrankungen durch somatische Gentherapie zu behandeln, wobei der Anteil mutierter mitochondrialer DNA (mtDNA) entweder unter den für die Ausprägung klinischer Symptome notwendigen Schwellenwert gedrückt oder die mutierte mtDNA vollständig entfernt wird.

Die menschliche mitochondriale DNA bzw. mtDNA ist eine ringförmige Doppelhelix aus 16 595 Basenpaaren (bp) und liegt in einer *supercoiled*-Konformation vor. Die Sequenz ist seit 1981 bereits vollständig aufgeklärt (Anderson, S. et al., Nature, 1981, 290: 457-465) und beinhaltet die Sequenz für 37 Gene. Dreizehn Gene davon codieren Polypeptide, die integrale Bestandteile von vier der fünf Atmungskettenkomplexe sind. Sieben dieser Gene (ND1 bis ND6 sowie ND4L) codieren für Untereinheiten der NADH-Ubichinon-Oxidoreduktase (Komplex I) und eines (CYTB) für Cytochrom b, einen Bestandteil der Ubichinol-Cytochrom c-Oxidoreduktase (Komplex III). Außerdem liegen drei Gene (COX1 bis COX3) für Untereinheiten der Cytochrom c-Oxidase (Komplex IV) sowie zwei Gene (ATP6/8) für Untereinheiten der ATP-Synthase (Komplex V) auf der mitochondrialen DNA vor. Daneben befinden sich auf dem Genom noch zwei ribosomale RNAs (rRNAs) und 22 Gene für transfer-RNAs (tRNAs), die für das mitochondriale Translations-System essentiell sind.

Die beiden DNA-Stränge der DNA-Doppelhelix werden aufgrund ihres unterschiedlichen Purin- und Pyrimidingehalts in einen schweren (H-Strang, *heavy*) und einen leichten Strang (L-Strang, *light*) unterschieden (Berk, A. J. und D. A. Clayton, J. Mol. Biol., 1974, 86: 801-824). Der Großteil der Information ist mit den Genen für zwei rRNAs, 14 tRNAs und zwölf Polypeptiden auf dem H-Strang codiert, während hingegen der L-Strang nur ein Protein- und acht tRNA-Gene trägt (Anderson, S. et al., Nature, 1981, 290: 457-465).

Im Gegensatz zum nukleären Genom ist das mitochondriale Genom extrem kompakt strukturiert. Neben dem Fehlen von Introns grenzen die Gene teilweise ohne jegliche nicht-codierende Sequenz direkt aneinander oder überlappen sogar, so dass manche Terminations-Codons erst nach der Transkription durch Polyadenylierung der mRNAs vervollständigt werden. Dabei sind die Protein- und rRNA-codierenden Gene meistens von einem tRNA-Gen flankiert. Insgesamt gibt es nur drei nicht-codierende Regionen, von denen der größte 1123 bp umfasst und als *Displacement-Loop* (*D-Loop*) bezeichnet wird (Anderson, S. et al., Nature, 1981, 290: 457-465). Die Bezeichnung *D-Loop* beschreibt eine der Hauptkonformationen der mitochondrialen DNA, die an dieser Stelle eine Tripel-DNA-Struktur aufweist. Sie enthält ein kurzes, neu synthetisiertes H-Strang-Molekül (7S-DNA), welches mit dem L-Strang Basenpaarungen eingeht (Tapper, D. P. und D. A. Clayton, J. Biol. Chem., 1981, 256: 5109-5115). Ferner stellt der *D-Loop* einen Bereich dar, der für die Regulation der Replikation und Transkription des mitochondrialen Genoms verantwortlich ist. In ihm liegen der Startpunkt der Replikation bzw. der Replikationsursprung (ori) des H-Stranges (O_{H}), konservierte Sequenzblöcke (CSB I-III), Terminations-assoziierte Sequenzen (TAS) sowie die Promotoren für die Transkription mit der Bezeichnung HSP1 (*heavy strand Promotor* 1, H1-Promotor) und LSP (*light strand Promotor*), und HSP2 (*heavy strand Promotor* 2, H2-Promotor), die sich hinsichtlich ihrer Transkriptionsraten unterscheiden, wobei der H1-Promotor eine 20-30-fach höhere Transkriptionsrate als der H2-Promotor besitzt und ungefähr doppelt so stark wie der L-Strang-Promotor ist (King, M. P. und G. Attardi, J. Biol. Chem., 1993, 268: 10228-10237)..

Der "mitochondriale Expressionsvektor" bzw. "Expressionsvektor" bzw. das "mitochondriale Expressionsplasmid" bzw. "Expressionsplasmid" muss somit, um seine Funktion erfüllen zu können, mindestens zwei Bestandteile aufweisen: exogene DNA, d.h. ein zu exprimierendes Gen, und einen mitochondrialen Promotorbereich. In einer bevorzugten Ausführungsform sind noch andere Bestandteile vorhanden, wie beispielsweise ein Selektionsmarker bzw. Reportergene, Signalsequenzen bzw. Transkriptions-Terminations-Sequenzen (TSS), Replikationsursprünge und/oder ein Sicherheitsmechanismus.

In einer bevorzugten Ausführungsform ist mit dem Begriff "mitochondrialer Expressionsvektor" bzw. "Expressionsvektor" bzw. "mitochondriales Expressionsplasmid" bzw. "Expressionsplasmid" ein komplettes mitochondriales Genom oder ein Derivat davon gemeint..

Die "exogene DNA" bzw. das zu "exprimierende Gen" stellt einen essentiellen Bestandteil des Expressionsvektors dar, der in den Expressionsvektor integriert wird, um beispielsweise eine somatische Gentherapie der oben bereits erwähnten mitochondrialen Erkrankungen zu ermöglichen. Dann stellt das zu exprimierende Gen ein "therapeutisches Gen" dar. Dabei umfasst der Begriff "therapeutisches Gen" ein intaktes replikatives und transkriptionsaktives Gen, um die mutierten Gene zu kompetitieren; ein intaktes Gen, um die mutierten Gene durch Rekombination auszutauschen; Gene, die für Antisense-Oligonukleotide codieren, um die Replikation der mutierten mtDNA zu inhibieren.

Die "mutierten Gene" umfassen beispielsweise die mutierten Formen von Genen der mtDNA, die Gene für die Pyruvatdehydrogenase, die Pyruvatcarboxylase, für die Proteine des Zitratzyklus, der Atmungskettenkomplexe I bis IV oder der ATP-Transportproteine oder Gene, die für Proteine oder Proteinkomplexe codieren, welche an der Regulation der mitochondrialen Replikation, Transkription oder der Translation beteiligt sind, umfassend beispielsweise die Gene für Transkriptionsfaktoren (TFAM, TFB1M, TFB2M), die mitochondriale RNA-Polymerase (POLRMT), mitochondriale Terminationsfaktoren (mTERF1-mTERF4), die mitochondriale DNA-Polymerase γ (POLG), das mitochondriale Einzelstrangbindeprotein mtSSB (*single strand binding*), die Helikase TWINKLE, mitochondriale Topoisomerasen vom Typ I und II, die mitochondrialen Peptidasen MIP (*mitochondrial intermediate peptidase*) und MPP (*mitochondrial processing peptidase*), das mitochondriale Hitzeschockprotein mtHSP70 (*mitochondrial heat shock protein*).

Die Veränderungen in den mutierten Genen der mtDNA der vorliegenden Erfindung werden durch Genmutationen hervorgerufen, die aus der Gruppe ausgewählt sind, die aus Punktmutationen durch Substitution, Deletionsmutationen durch Deletion, Insertionsmutationen durch Insertion und Duplikationsmutationen durch Duplikation besteht, insbesondere aus Deletionsmutationen oder Punktmutationen. Die Deletionen in den mutierten Genen sind zwischen 1,0 und 10 kb groß, insbesondere zwischen 1,3 und 8,0 kb groß, und befinden sich meistens zwischen den beiden mitochondrialen Replikationsursprüngen O_{H} und O_{L} und sind in der Regel von kurzen Repeatsequenzen flankiert. Im Gegensatz zu Deletionsmutationen befinden sich die Punktmutationen der mutierten Gene der mtDNA der vorliegenden Erfindung überall auf der mtDNA, z.B. in Proteingenen, tRNA- oder rRNA-Genen, insbesondere in den tRNA-Genen. Während Punktmutationen in Proteingenen nur einzelne Bestandteile der Atmungskette betreffen, wird durch Punktmutationen in den tRNA- und rRNA-Genen die gesamte mitochondriale Proteinbiosynthese und somit alle mitochondrial codierten Atmungskettenuntereinheiten beeinträchtigt.

Die "intakten Gene" umfassen somit die entsprechenden nicht-mutierten Formen der oben bereits aufgelisteten Gene, die für die Pyruvatdehydrogenase, die Pyruvatcarboxylase, für die Proteine des Zitratzyklus, der Atmungskettenkomplexe I bis IV oder der ATP-Transportproteine oder für Proteine oder Proteinkomplexe codieren, welche an der Regulation der mitochondrialen Replikation, Transkription oder der Translation beteiligt sind.

Ferner kann in den Expressionsvektor zusätzlich zum oder anstelle des zu exprimierenden Gens auch ein "Selektionsmarker" bzw. Marker- bzw. Reportergen integriert werden, um beispielsweise die Anwendung des mitochondrialen Expressionsvektors in der Grundlagenforschung zu ermöglichen. Der "Selektionsmarker" lässt die Unterscheidung zwischen transfizierten Zellen und nichttransfizierten Zellen, insbesondere lebender Zellen, zu. Dabei umfasst der Selektionsmarker die kodierende Sequenz oder das Gen, das für ein fluoreszierendes Protein oder für ein Reporterenzym codiert, oder ein Antibiotika-Resistenzgen.

Bevorzugte fluoreszierende Proteine sind z.B. das grün fluoreszierende Protein (GFP), das blau fluoreszierende Protein (BFP), das cyan fluoreszierende Protein (CFP) oder das gelb fluoreszierende Protein (YFP) oder Derivate davon, wobei die Derivate aus der Gruppe ausgewählt sind, die aus dem 25-kDa *enhanced GFP* (EGFP), Derivaten des blau fluoreszierenden Proteins (z.B. EBFP, EBFP2, Azurit oder mKalama1), Derivaten des cyan fluoreszierenden Proteins (z.B. ECFP, Cerulean oder CyPet) und Derivaten des gelb fluoreszierenden Proteins (z.B. Citrin, Venus oder YPet) besteht, wobei EGFP das bevorzugteste Derivat darstellt.

Bevorzugte Reporterenzyme bzw. Gene, die für ein Reporterenzym codieren sind: z.B. das *Lac*Z-Gen aus *Escherichia coli*, codiert für eine β-Galactosidase (β-Gal); das *pho*A-Gen aus *Escherichia coli*, codiert für eine Alkalische Phosphatase (AP); das *gus*A-Gen aus *Escherichia coli*, codiert für eine β-Glucuronidase (GUS) (früher bekannt als uidA-Gen); das *cat*-Gen, codiert für eine Chloramphenicol Acetyltransferase (CAT); das *Luciferase*-Gen aus *Photinus pyralis* und *Renilla reniformis*, codiert für das Biolumineszenz-Enzym Luciferase, vorzugsweise für die Firefly-Luciferase.

Bevorzugte Antibiotika-Resistenzgene sind z.B. das Ampicillin-Resistenzgen ampR (auch bekannt unter der Bezeichnung blaTEM1), das Tetrazyclin-, das Kanamycin-, Neomycin-Resistenzgen oder das Chloramphenicol-Resistenzgen (Cam^{R}), wobei das Chloramphenicol-Resistenzgen das Bevorzugteste ist.

In einer bevorzugten Ausführungsform der vorliegenden Erfindung werden in den Expressionsvektor mehr als ein Selektionsmarker, z.B. zwei Selektionsmarker integriert, beispielsweise die codierende Sequenz für EGFP und das Chloramphenicol-Resistenzgen. In einer weiteren bevorzugten Ausführungsform werden der/die Selektionsmarker zusammen mit dem zu exprimierenden Gen in den Expressionsvektor integriert.

In einer spezifischen Ausführungsform der Erfindung sind an beide Seiten des jeweiligen integrierten Gens, z.B. des zu exprimierenden Gens bzw. Selektionsmarkers, "flankierende tRNAs" fusioniert, um die korrekte Prozessierung eigener Genkonstrukte zu gewährleisten. Diese tRNAs markieren nach der Transkription durch Ausbildung ihrer klassischen Kleeblattstrukturen als "Erkennungsstellen" für die Prozessierungsenzyme die Prozessierungsstellen, indem sie als Sekundärstruktur ihre klassische Kleeblattstruktur einnehmen und werden dann aus dem Transkript mittels Restriktionsspaltung herausgeschnitten. Dadurch werden die RNAs der flankierten Genbereiche freigesetzt und können anschließend weitergehend modifiziert werden, z.B. Polyadenylierung der mRNAs und rRNAs am 3'-Ende durch eine mitochondriale Poly(A)-Polymerase. Die "flankierenden tRNAs" können alle mitochondrialen und nukleären tRNAs sein, vorzugsweise die tRNAs bzw. tRNA-Paare für Glycin und Arginin, Leucin und Valin, Aspartat und Lysin umfassen.

Neben dem zu exprimierenden Gen und/oder dem Selektionsmarker weist der Expressionsvektor noch einen weiteren essentiellen Bestandteil auf: einen mitochondrialen Promotorbereich. Unter dem Begriff "mitochondrialer Promotorbereich" ist gemäß der vorliegenden Erfindung der D-Loop des mitochondrialen Genoms gemeint, der die für die Transkriptionsinitiation wichtigen Sequenzen enthält. Dazu zählen die drei unabhängigen mitochondrialen Promotoren für den L- bzw. H-Strang: HSP1, HSP2 und/oder LSP, der Startpunkt der Replikation bzw. Replikationsursprung des H-Stranges (O_{H}), konservierte Sequenzblöcke (CSB I-III), sowie Terminations-assoziierte Sequenzen (TAS).

Der mitochondriale Promotorbereich ermöglicht die regulierte Expression eines Gens, das in den Expressionsvektor der vorliegenden Erfindung integriert worden ist, wobei RNA-Polymerasen die Enzyme sind, die die Synthese von RNA bei der Transkription der DNA katalysieren. Die Transkription von Säuger- mtDNA erfolgt normalerweise durch eine "mitochondriale RNA-Polymerase" (POLRMT oder mtRNApol), die eine hohe Sequenzähnlichkeit zu RNA-Polymerasen aus Bakteriophagen aufweist (Masters, B. S. et al., Cell, 1987, 51: 89-99). Allerdings gehört das mitochondriale RNA-Polymerase-Gen nicht zu den essentiellen Bestandteilen, die in den Expressionsvektor zu integrieren sind, so dass im Rahmen der vorliegenden Erfindung normalerweise die bereits vorhandene mitochondriale RNA-Polymerase des transfizierten Mitochondriums verwendet wird. Da POLRMT allerdings nicht die Fähigkeit besitzt, selbstständig mit der Promotor-DNA zu interagieren und so die Transkription zu initiieren, sondern dafür zusätzliche trans-aktive Faktoren bzw. Transkriptionsfaktoren benötigt, wie beispielsweise der mitochondriale Transkriptionsfaktor A (TFAM, mtTFA), B1 (TFB1 M) oder B2 (TFB2M), werden im Rahmen der vorliegenden Erfindung neben der mitochondrialen RNA-Polymerase des transfizierten Mitochondriums ebenfalls die Transkriptionsfaktoren des transfizierten Mitochondriums verwendet. Diese Transkriptionsfaktoren binden in der Nähe der Promotoren an die mitochondriale DNA und ermöglichen dadurch die Bindung von POLRMT, die dann mit der Transkription beginnen kann.

In einer weiteren bevorzugten Ausführungsform kann neben der mitochondrialen RNA-Polymerase noch eine weitere RNA-Polymerase genutzt werden, um die Expressionsrate z.B. des zu exprimierenden Gens und/oder des Selektionsmarkers zu verstärken, z.B. die hoch-prozessive RNA-Polymerase aus dem Bakteriophagen T7 ("T7-RNA-Polymerase"), die RNA-Polymerase aus dem Bakteriophagen T3 ("T3-RNA-Polymerase") und die RNA-Polymerase aus dem Bakteriophagen SP6 ("SP6-RNA-Polymerase"). Das Gen, das für die T7-RNA-Polymerase codiert, ist in dieser Ausführungsform in den Expressionsvektor zu integrieren und unter die Kontrolle des mitochondrialen Promotors zu stellen. Um die Transkriptionsverstärkung durch die T7-RNA-Polymerase zu nutzen, ist in dieser weiteren bevorzugten Ausführungsform das zu exprimierende Gen noch mit dem T7-Promotor zu versehen, der neben den H- und L-Strang-Promotoren im Expressionsvektor integriert sein kann. Dadurch steht die T7-RNA-Polymerase vorzugsweise unter der Kontrolle der mitochondrialen Promotoren, wobei das zu exprimierende Gen und/oder der Selektionsmarker durch die hoch-prozessive T7-RNA-Polymerase produziert werden.

In einer bevorzugten Ausführungsform ist im Falle eines weiteren Promotors neben den H- und L-Strang Promotoren, z.B. im Falle des integrierten T7-Promotors, eine "Signalsequenz" bzw. "Transkriptions-Terminations-Sequenz" (TTS) im Expressionsvektor vorhanden, die verhindert, dass die T7-Promotor-gesteuerte Expression des z.B. zu exprimierenden Gens und/oder des Selektionsmarkes durch die mitochondrialen Transkriptionsvorgänge behindert wird. Die TTS der vorliegenden Erfindung befindet sich vorzugsweise hinter dem Sequenzbereich des zu exprimierenden Gens und/oder des Selektionsmarkes, um die Transkription zu stoppen. In einer bevorzugten Ausführungsform der vorliegenden Erfindung stellt eine der flankierenden tRNAs die TTS dar, vorzugsweise die flankierende tRNA für Leucin, die die vom mitochondrialen Promotor, z.B. HSP1, ausgehende Transkription stoppt, wodurch die Expression z.B. des zu exprimierenden Gens und/oder des Selektionsmarkers verstärkt wird.

Eine weitere Verstärkung der Expression gemäß der vorliegenden Erfindung ist dadurch erreichbar, dass eine Vermehrung bzw. Replikation des Expressionsvektors innerhalb der Mitochondrien ermöglicht wird, da dann nach einer Zellteilung der Expressionsvektor an beide Tochterzellen weitergegeben wird, was eine dauerhafte Expression erlaubt. Die mtDNA wird mittels einer eigenen Replikationsmaschinerie dupliziert, wobei ein im Stand der Technik angenommenes Modell beschreibt, dass die mitochondriale Replikation von zwei Replikationsursprüngen aus asymmetrisch verläuft (z.B. Shadel, G. S. und D. A. Clayton, Annu. Rev. Biochem., 1997, 66: 409-435).

Beim asymmetrischen Modell beginnt die Replikation des H-Stranges am O_{H}, der stromabwärts des LSP liegt. Der für die Initiation benötigte Primer wird durch die vom LSP ausgehende Transkription gebildet. Das dabei entstehende RNA-Fragment bleibt über die konservierten Sequenzblöcke (CSB I-III) an die DNA gebunden. Zusammen mit dem verdrängten H-Strang bildet dieses RNA/DNA-Hybrid eine stabile dreisträngige Struktur, die als R-Loop bezeichnet wird. Der RNA-Strang wird durch eine Endonukleaseaktivität an bestimmten Stellen in der O_{H}-Region gespalten und kann daraufhin als Primer für die DNA-Synthese des H-Stranges durch die mitochondriale DNA-Polymerase y (POLG) dienen. Die Replikation des H-Stranges bricht in den meisten Fällen im Bereich der Terminations-assoziierten Sequenzen (TAS, 700 bp stromabwärts des O_{H}) ab. Durch den Terminationsvorgang entsteht die sogenannte 7S DNA, die zusammen mit den beiden Strängen der mtDNA die charakteristische dreisträngige Struktur des *D-Loop* ergibt. Wird jedoch die H-Strang-Synthese weitergeführt, erreicht sie nach 2/3 der Elongation den O_{L}, wodurch die Replikation des L-Stranges in entgegengesetzter (asymmetrischer) Richtung beginnt. Die verdrängte einzelsträngige DNA am O_{L} bildet eine *stem-loop*-Struktur aus, die zur Rekrutierung einer möglichen mitochondrialen Primase führt. Diese synthetisiert in der thymidinreichen Region des *Loops* den RNA-Primer für die Replikation des L-Stranges. Im Bereich einer Pentanukleotidsequenz an der Basis der Sekundärstruktur erfolgt der Übergang zwischen RNA- und DNA-Synthese. Sind beide Stränge repliziert, werden die RNA-Primer entfernt, Lücken aufgefüllt sowie ligiert und superhelikale Windungen in die geschlossenen zirkulären mtDNA-Moleküle eingeführt (z.B. Shadel, G. S. und D. A. Clayton, Annu. Rev. Biochem., 1997, 66: 409-435).

Somit weisen die erfindungsgemäß eingesetzten Expressionsvektoren , die lediglich den Replikationsursprung für den H-Strang, der im mitochondrialen Promotorbereich integriert ist, beinhalten, den Nachteil auf, dass sie in den Mitochondrien nicht repliziert werden, wodurch nach einer Zellteilung die Weitergabe des Expressionsvektors an beide Tochterzellen verhindert wird und somit eine dauerhafte Expression nicht möglich ist. Insofern wird in einer spezifischen Ausführungsform der vorliegenden Erfindung zusätzlich der Replikationsursprung für den L-Strang in den Expressionsvektor integriert, vorzugsweise hinter das zu exprimierende Gen und/oder den Selektionsmarker, um die genannten Nachteile zu umgehen und so die Expression zu verstärken.

Ein weiterer Mechanismus kann in den Expressionsvektor eingebaut sein, um die Expression z.B. des zu exprimierenden Gens und/oder des Selektionsmarkers nach einer möglichen Integration ins Kerngenom zu verhindern. Dazu wird z.B. das zu exprimierende Gen und/oder der Selektionsmarker so verändert, dass es/sie nach der Translation in den Mitochondrien funktionell ist/sind, während eine Translation im Zytosol ein nicht funktionsfähiges Protein entstehen lässt. Hierzu werden in einer bevorzugten Ausführungsform "Sicherheitsmechanismen" eingebaut, die darauf basieren, dass die mtDNA im Vergleich zum Kerngenom einige Unterschiede in der Codon-Nutzung aufweisen.

Zusätzlich lässt sich die Translation durch den Einsatz "Codonusage"-optimierter Gene verbessern. Beispielsweise ist in dieser bevorzugten Ausführungsform ein Kern-Stop-Codon, vorzugsweise TGA, im mitochondrialen Expressionsvektor z.B. im zu exprimierenden Gen und/oder im Selektionsmarker integriert, dass in den Mitochondrien jedoch für Tryptophan codiert. Somit wird bei einer Translation des z.B. zu exprimierenden Gens und/oder des Selektionsmarkers im Zytosol ein Abbruch initiiert und das Protein in einer nicht aktiven Form erhalten.

Folglich weist der "mitochondriale Expressionsvektor" bzw. "Expressionsvektor" mindestens die zwei Bestandteile auf: exogene DNA, d.h. ein zu exprimierendes Gen und/oder einen Selektionsmarker, und einen mitochondrialen Promotorbereich. Erst in bevorzugten Ausführungsformen sind noch andere Bestandteile vorhanden, wie beispielsweise weitere Selektionsmarker, Signalsequenzen bzw. Transkriptions-Terminations-Sequenzen (TTS), Replikationsursprünge und/oder ein Sicherheitsmechanismus. Bevor die exogene DNA, die in dem mitochondrialen Expressionsvektor integriert, mittels einer physikalischen Transfektionsmethode in die Mitochondrien von Säugerzellen eingebracht wird, werden die Mitochondrien zuvor reversibel zu Megamitochondrien induziert, da Mitochondrien aufgrund ihrer geringen Dicke von 0,5-1 µm nur schwer transfiziert werden können.

Mitochondrien, die gegenüber normalen Mitochondrien erheblich vergrößert sind, so dass sie im Lichtmikroskop ohne Anfärben zu erkennen sind, werden gemäß der vorliegenden Erfindung als "Megamitochondrien" bzw. "Riesenmitochondrien" bezeichnet. Diese zeichnen sich insbesondere dadurch aus, dass sie angeschwollen sind (PCT/EP2009/004454), wobei sie vorzugsweise einen Durchmesser von ≥ 2 µm, bzw. von 2-10 µm, bevorzugt von 2, 3, 4, 5, 6, 7, 8, 9 oder 10 µm, am bevorzugtesten zwischen 4-10 µm oder 4, 5, 6, 7 oder 10 µm aufweisen.

Die reversible Induktion der Megamitochondrien erfolgt vor der physikalischen Transfektion der Mitochondrien, wobei gemäß der vorliegenden Erfindung die Megamitochondrien entweder durch die Gabe von Wirkstoffen bzw. Chemikalien in das Kulturmedium, vorzugsweise Valinomycin (Malka, F. et al., EMBO Rep., 2005, 6: 853-859), Chloramphenicol (Albring, M. et al., Naturwissenschaften, 1975, 62: 43-44), Cuprizon (Suzuki, K., Science, 1969, 163: 81-82) oder H₂O₂ oder Hydrazin (Teranishi, M. et al., J. Electron. Microsc., (Tokyo), 1999, 48: 637-651), mehr bevorzugt Valinomycin, oder durch die Inkubation der Zellen im sauren Kulturmedium reversibel induziert werden, wobei das Kulturmedium durch Zugabe oder durch Entstehenlassen von pH-Wert senkenden Substanzen, beispielsweise Säuren oder deren Salze, angesäuert bzw. azidifiziert wird (PCT/EP2009/004454), vorzugsweise durch Zugabe oder Entstehenlassen von Milchsäure, Lactat, Essigsäure und/oder Natriumacetat bzw. Gemischen davon.

Unter dem Begriff "Kulturmedium" bzw. "Nährmedium" wird gemäß der vorliegenden Erfindung das Substrat gemeint, das zur Kultur der Zellen dient. Die für die vorliegende Erfindung geeigneten Kulturmedien sind beispielsweise das Iscove's Medium, RPMI Medium, Dulbecco's MEM Medium, MEM Medium, F12 Medium, Dulbecco's modified Eagle's medium (DMEM) oder Eagle's Minimum Essential Medium (EMEM). Diese Medien können mit Zusatzstoffen angereichert werden, wie beispielsweise mit fetalem Kälberserum, Bromdesoxyuridin, Pyruvat, Uridin, Earle's Salze, L-Glutamin, Natriumbicarbonat oder nicht-essentiellen Aminosäuren.

"Valinomycin" ist ein K⁺-selektives Zyklosidpeptid, welches als lonophor agiert und die oxidative Phosphorylierung entkoppelt. Zur reversiblen Induktion von Megamitochondrien sind die Zellen vor der physikalischen Transfektion der Mitochondrien für 1-5 h in Kulturmedium mit 1-20 µM Valinomycin zu inkubieren, vorzugsweise für 1, 2 oder 4 h in Kulturmedium mit 10 µM Valinomycin.

Neben der reversiblen Induktion der Megamitochondrien durch die Zugabe von Chemikalien, ist auch die Inkubation der Zellen in saurem Kulturmedium eine Möglichkeit, Mitochondrien zum Schwellen zu bringen. Das Kulturmedium ist hierfür durch Zugabe oder durch Entstehenlassen pH-Wert senkender Substanzen, beispielsweise Säuren oder Salzen davon, auf einen pH-Wert von zwischen 5,3 und 6,7, vorzugsweise von zwischen 5,7 und 6,3 oder von zwischen 6,4 und 6,5 anzusäuern.

Die Ansäuerung mittels pH-Wert senkender Substanzen kann zum einen durch die Zugabe von Milchsäure oder ihrer Salze, beispielsweise Lactat, erfolgen, so dass die finale Konzentration von Milchsäure oder des Salzes im Kulturmedium vorzugsweise bei zwischen 5 bis 100 mM, vorzugsweise bei zwischen 40 bis 70 mM liegt. Das erzeugt einen pH-Wert im Kulturmedium von zwischen 5,3 und 7,0, vorzugsweise von zwischen 5,3 bis 6,7, bevorzugt von zwischen 5,7 und 6,3 oder von zwischen 6,4 und 6,5, am bevorzugtesten einen pH-Wert von 6,3 (siehe PCT/EP2009/004454).

Weitere Säuren, die als pH-Wert senkende Substanzen zur Ansäuerung des Kulturmediums im Rahmen der vorliegenden Erfindung verwendet werden können, umfassen beispielsweise Essigsäure und ihre Salze. Die Zugabe von Essigsäure oder ihrer Salze erfolgt so, dass die ihre finale Konzentration im Kulturmedium bei zwischen 5 bis 100 mM, vorzugsweise bei zwischen 25 bis 40 mM, mehr bevorzugt bei zwischen 30 bis 35 mM liegt. Das erzeugt einen pH-Wert im Kulturmedium von zwischen 5,3 bis 7,0, vorzugsweise von 5,7 bis 7,0, bevorzugt von zwischen 6,3 und 7,0, am bevorzugtesten einen pH-Wert von 6,3

Im Hinblick auf die Essigsäure kann insbesondere ihr Natriumsalz Natriumacetat als pH-Wert senkende Substanz verwendet werden, wobei dessen Zugabe so erfolgt, dass die finale Konzentration bei zwischen 5 bis 100 mM, vorzugsweise bei zwischen 45 bis 65 mM, vorzugsweise bei 45, 50, 55, 60 oder 65 mM, am bevorzugtesten bei zwischen 50 oder 60 mM liegt. Das erzeugt einen pH-Wert im Kulturmedium von zwischen 5,3 bis 7,0, vorzugsweise von zwischen 5,7 bis 7,0, bevorzugt von zwischen 6,3 und 7,0, am bevorzugtesten einen pH-Wert von 6,3.

Ferner kann die Ansäuerung auch durch Stoffwechselprodukte erfolgen, die durch Kultivierung der Zellen ohne Medienwechsel im Kulturmedium entstehen, d.h. die Ansäuerung kann durch "Entstehenlassen von pH-Wert senkenden Substanzen" im Kulturmedium erfolgen, beispielsweise Lactat, das beim Abbau von Zuckern durch die Milchsäuregärung als Stoffwechselzwischenprodukt in einer Konzentration von zwischen 15 bis 50 g/I, vorzugsweise von zwischen 25 bis 30 g/I entsteht und somit von den Säugerzellen selbst produziert wird und einen pH-Wert von zwischen 6,3 und 7,0, vorzugsweise von zwischen 6,7 und 6,8 erzeugt.

Die Säugerzellen werden zur reversiblen Induktion der Megamitochondrien gemäß der vorliegenden Erfindung im angesäuerten Medium inkubiert, vorzugsweise für zwischen 10 min und 5 Tage, vorzugsweise für zwischen 20 min und 2 Tage, mehr bevorzugt für 20 min, 60 min und 2 Tage. Die Inkubation erfolgt vorzugsweise bei 37°C, einer relativen Luftfeuchtigkeit von 95% und 5% CO₂-Begasung.

Unter dem Begriff "Säugerzellen" bzw. "Zellen" sind gemäß der vorliegenden Erfindung Zellen von Säugerzelllinien gemeint, wobei "Säugerzelllinien" die Zelllinien 143B.TK⁻, 143B.TK⁻+pEGFP-Mito, 143B.TK⁻+pEGFP-OMP, 143B.TK⁻K7 und He-Ia296-1 umfassen. Zum Zeitpunkt der reversiblen Induktion der Megamitochondrien wiesen die Säugerzellkulturen eine Zelldichte von zwischen 1-5 x 10⁵ Zellen pro 35 mm-Schale, vorzugsweise von 3 x 10⁵ Zellen pro 35 mm-Schale. Umgerechnet bedeutet dies ca. 1-5 x 10⁴ Zellen pro cm². In einer weiterenAusführungsform umfasst der Begriff "Säugerzellen" bzw. Zellen" gemäß der vorliegenden Erfindung "rho⁰-Zellen" bzw. "ρ⁰-Zellen" (siehe PCT/EP2008/010586), die für erfindungsgemäße Transfektionsversuche mit mtDNA verwendet werden, bei denen komplette mitochondriale Genome oder Teile/Derivate davon aufzunehmen sind.

Die Induktion der Megamitochondrien, die gemäß der vorliegenden Erfindung durchgeführt wird, zeichnet sich dadurch aus, dass sie reversibel ist, wobei unter dem Begriff "reversibel" bzw. "Reversibilität" die Umkehrung zu verstehen ist, dass sich die durch Induktion hergestellten Megamitochondrien in den Zustand vor ihrer Induktion ohne Veränderungen zurückbilden können. Hierfür wird das mit Wirkstoffen angereicherte bzw. angesäuerte Kulturmedium jeweils mit einem Kulturmedium ausgetauscht, das entweder nicht mit Wirkstoffen, z.B. nicht mit pH-Wert senkenden Substanzen angereichert oder nicht angesäuert ist, d.h. vorzugsweise einen pH-Wert von ≥ 7,0, vorzugsweise einen pH-Wert ≥ 7,4 aufweist.

Die Methode für die Induktion der Megamitochondrien bzw. die Auswahl des für die Anreicherung verwendeten Wirkstoffes bzw. der für die Ansäuerung verwendeten Säure oder Salzes erfolgt in Abhängigkeit von der Transfektionsmethode. Unter dem Begriff "Transfektion" ist gemäß der vorliegenden Erfindung das Einbringen von Fremd-DNA in eukaryontische Zellen zu verstehen, wodurch der Zelle erlaubt wird, fremde bzw. heterologe Gene nach der DNA-Aufnahme zu exprimieren. Man unterscheidet zwischen zwei Arten der Transfektion: Bei der "transienten Transfektion" wird ein Vektor in die Wirtszelle eingebracht, um eine vorübergehend starke Expression eines heterologen Gens zu erreichen. Diese Expression geht jedoch mit der Verdünnung des Vektors bei jeder Zellteilung rapide zurück. Sehr selten kommt es durch illegitime Rekombination zu einer Integration des Vektors in das Genom der Zelle, so dass die Verdünnung nicht mehr fortschreitet. Bei der "stabilen Transfektion" wird durch den dauerhaften Einbau der Vektor-DNA in das Wirtsgenom eine langfristige Genexpression anvisiert. Voraussetzung ist allerdings, dass ein selektierbares Gen mittransfiziert wird. Werden retrovirale Expressionsvektoren eingesetzt, ist eine stabile Integration der Vektor-DNA gewährleistet.

Gemäß der vorliegenden Erfindung ist eine physikalische Transfektionsmethode zu verwenden, wobei die physikalische Transfektionsmethode aus der Gruppe ausgewählt ist, die aus dem Beschießen der Zellen mit DNA-beschichteten Mikropartikeln in einer Genkanone, der Transfektion mithilfe magnetischer Partikel und der Mikroinjektion besteht.

Das Beschießen der Zellen mit DNA-beschichteten Mikropartikeln, vorzugsweise Gold- oder Wolframpartikeln, erfolgt gemäß der vorliegenden Erfindung in einer Genkanone (*Gene Gun*), wobei es sich hierbei um ein biolistisches, d.h. um ein rein mechanisches, Verfahren handelt, DNA mit Hilfe von Partikeln in Zellen zu schießen. Die DNA wird dabei auf die Oberfläche der Partikel aufgebracht, die anschließend unter hohem Druck auf die Säugerzelle geschossen werden. Am Zielort, d.h. in den Mitochondrien, wird die DNA anschließend freigesetzt und kann dort exprimiert werden. Im Rahmen der vorliegenden Erfindung wurden spezifische Parameter eingestellt, um eine Transfektionsrate von bis zu 8% zu erreichen. Die Mikropartikel (Wolfram- oder Goldpartikel, vorzugsweise Goldpartikel) besitzen einen Durchmesser von zwischen 0,2 bis 5 µm, vorzugsweise von 0,6 bis 2,5 µm, bevorzugt von 0,6; 1,0, 1,2 oder 1,6 µm, mehr bevorzugt von 0,6 µm, wobei pro Transfektionsansatz zwischen 0,1 bis 5 µg, vorzugsweise zwischen 0,5 bis 2 µg, bevorzugt 1 µg des mitochondrialen Expressionsplasmids und 0,05 bis 2,5 µg, vorzugsweise 0,1 bis 1,0 µg, vorzugsweise 0,5 µg Partikel zu verwenden sind.

Das Beschießen der Zellen mit DNA-beschichteten Partikeln hat gemäß der vorliegenden Erfindung bei Kammerdrücken zwischen 5 bis 50 in Hg, vorzugsweise zwischen 15 bis 27 in Hg, vorzugsweise bei 15, 20, 25 und 27 in Hg, mehr bevorzugt bei ≥ 25 in Hg, am bevorzugtesten bei 27 in Hg Unterdruck oder im Vakuum, unter Verwendung von 100-3000 psi, vorzugsweise von 1350-2000 psi, bevorzugt von 1350, 1550, 1800 oder 2000 psi, mehr bevorzugt von 1800 psi Berstscheiben (*Rupture Disks*); und/oder auf einer der Einschubebenen A-D der Genkanone, wobei A die oberste und D die unterste Einschubebene darstellt, vorzugsweise auf der zweitobersten Einschubebene (Ebene B) oder der zweituntersten Ebene (Ebene C), bevorzugt auf der zweituntersten Ebene (Ebene C) der Genkanone zu erfolgen, wobei die Zellen für den Beschuss in Kunststoff-Kulturschalen mit einem Durchmesser von 35-150 mm, vorzugsweise von 35, 60, 100 oder 150 mm, bevorzugt von 35 kultiviert werden sollten. Das Aufbringen der DNA auf die Mikropartikel geschieht gemäß der vorliegenden Erfindung dadurch, dass die DNA im Beisein der Partikel vorzugsweise durch Neutralsalze, vorzugsweise durch CaCl₂ ausgefällt wird, wobei dieser Vorgang durch die Zugabe von 0,05-0,2 M, vorzugsweise von 0,1 M Spermidin noch verstärkt werden kann.

Neben dem Beschießen der Zellen mit DNA-beschichteten Mikropartikeln in einer Genkanone kann die physikalische Transfektion der Megamitochondrien noch mithilfe magnetischer Partikel erfolgen. Bei dieser Methode werden magnetische Partikel, vorzugsweise magnetische Nanopartikel, z.B. MagTag^{™}, genutzt, um DNA in die Zellen zu transportieren, wobei die magnetischen Partikel mit DNA beschichtet, über den Zellen im Kulturmedium verteilt und durch eine Magnetplatte in die Zellen gezogen werden, wo sie die DNA freigeben. Diese Methode hat den Vorteil, dass die Zellmembran nicht durch chemische Reagenzien beeinflusst wird. Hierfür werden Transfektionsreagenzien verwendet, die die magnetischen Nanopartikel beinhalten, z.B. MaTra-A (IBA, Göttingen, Deutschland).

Eine weitere physikalische Transfektionsmethode stellt die Transfektion durch Mikroinjektion von DNA dar, bei der eine Glaskapillare durch die Zellmembran geschoben wird, durch die anschließend unterschiedliche Nukleinsäuren, z.B. der mitochondriale Expressionsvektor bzw. das mitochondriale Expressionsplasmid, in die Zelle eingebracht werden kann.

Für die Mikroinjektion gemäß der vorliegenden Erfindung sind die Zellen auf Glasbodenschalen mit integriertem Raster auszusäen, um die injizierten Zellen später leichter auffinden zu können. Nach der Induktion von Megamitochondrien sind die Säugerzellen an einem Mikromanipulator zu injizieren, wozu die Injektionskapillare mit 10-500 ng/µl, vorzugsweise mit 50-350 ng/µl DNA-Lösung enthaltend das mitochondriale Expressionsplasmid befüllt, an die Apparatur anzuschließen und mit dem Mikromanipulator an die zu injizierenden Säugerzellen heranzuführen ist. Die eigentliche Injektion erfolgt direkt axial in die Megamitochondrien mit einem Injektionsdruck von 50-150 hPa, vorzugsweise von 90-120 hPa, einer Injektionszeit von 0,1-0,2 s und/oder einem Haltedruck von 40-50 hPa.
Die Mikroinjektion stellt die bevorzugteste physikalische Transfektionsmethode der vorliegenden Erfindung dar Methode, da bei der Mikroinjektion sehr große Mitochondrien (bevorzugt >3 µm) verwendet werden und man somit optisch verfolgen kann, ob die Mitochondrien wirklich transfiziert bzw. penetriert wurden.

In einer spezifischen Ausführungsform der vorliegenden Erfindung werden vor dem Beschießen der Säugerzellen mit DNA-beschichteten Goldpartikeln in einer Genkanone oder vor der Transfektion mithilfe magnetischer Partikel die Megamitochondrien durch Ansäuerung des Kulturmediums mittels Milchsäure und vor der Mikroinjektion der Säugerzellen die Megamitochondrien durch Ansäuerung des Kulturmediums mittels Zugabe von Natriumacetat oder Essigsäure induziert.

Folglich ist der Gegenstand der vorliegenden Erfindung zudem ein Verfahren zum Einbringen exogener DNA in Mitochondrien von Säugerzellen, das die folgenden Schritte umfasst:
(i) Konstruktion eines mitochondrialen Expressionsvektors,
(ii) reversible Induktion von Megamitochondrien und
(iii) Transfektion der Megamitochondrien mit dem mitochondrialen Expressionsvektor mittels einer physikalischen Transfektionsmethode.

Die folgenden Beispiele dienen der Veranschaulichung des Gegenstands der vorliegenden Erfindung.

### Beispiel 1: Mikrobiologische Methoden

### Flüssigkulturen von Bakterien

### Benötigtes Material:

LB-Medium
Antibiotikum-Stammlösung

### Durchführung:

Für eine Flüssigkultur wurden je nach benötigter Menge 10 ml LB-Medium in einem 50-ml-Röhrchen oder 5 ml in einem 15-ml-Röhrchen vorgelegt. Bei Bedarf wurde zur Selektion des gewünschten Bakterienstammes das benötigte Antibiotikum (Tab. 1) hinzugefügt. Das Medium wurde mittels einer sterilen Pipettenspitze mit einer gepickten Kolonie von einer Agar-Platte und über Nacht bei 37°C unter Schütteln inkubiert.

**Tabelle 1: Eingesetzte Antibiotika-Konzentrationen**

| **Antibiotikum** | **Konzentration der Stocklösung** | **gelöst in** | **Stocklösung/ml Medium** | **Endkonzentration im Medium** |
|---|---|---|---|---|
| Ampicillin | 50 mg/ml | ddH₂O | 2-3 µl | 100-150 µg/ml |
| Kanamycin | 10 mg/ml | ddH₂O | 5 µl | 50 µg/ml |
| Tetracyclin | 5 mg/ml | Ethanol | 10 µl | 50 µg/ml |
| Chloramphenicol | 34 mg/ml | Ethanol | 0,5-5 µl | 17-170 µg/ml |

### Beispiel 2: Gewinnung mitochondrialer DNA aus humaner Placenta (modifiziert nach Fernandez-Silva, P. et al., Methods Enzymol., 1996, 164, 129-139)

Humane mitochondriale DNA kann in größeren Mengen aus Placenta präpariert werden. Dies ist der einfachste Weg, da andere humane Gewebe nur schwer zugänglich sind, während Placenten bei jeder Geburt anfallen. Die Präparation beruht hauptsächlich auf einer differenziellen Zentrifugation, bei der die Mitochondrien vom Kern und Zelltrümmern gereinigt werden und anschließend die DNA aus ihnen präpariert wird.

### Benötigtes Material:

Sucrose-TE-Puffer (STE)
Sucrose-TE-Puffer+ (STE+)
Sucrose-TE-Puffer mit MgCl₂ (STEM)
0,5 M EDTA
5 M NaCl
0,5 M SDS
DNase I (RNase-frei)
RNase A (DNase-frei)
Verbandmull
Miracloth
Glas-Homogenisator
Corex-Glas-Zentrifugenröhrchen

### Durchführung:

Nach der Abholung aus dem Kreißsaal wurde die Placenta auf Eis abgekühlt. Anschließend wurde die Placenta in kleine Stücke geschnitten, wobei das Bindegewebe und größere Gefäße abgetrennt wurden. Die Stücke wurden dann solange in STE gewaschen, bis nahezu kein Blut mehr austrat. Nach dem Wiegen (normalerweise 300-350 g) wurden die Stücke bis zur weiteren Verarbeitung in Form einer dünnen Platte bei -80°C eingefroren. Nach dem Antauen für ca. 1 h bei RT folgte die Zerkleinerung (durch Brechen) der Platte in kleine Stücke, die dann für 1,5 h langsam in 1 I STE gerührt wurden, bis sie völlig aufgetaut waren. Nach dem Überführen der Stücke in 600 ml neuen STE erfolgte die Zerkleinerung im Mixer für 3 × 20 s bei niedriger Geschwindigkeit. Nach einer Zentrifugation für 15 min bei 500 × g und 4°C zur Entfernung der Zelltrümmer wurden die Überstände durch zwei Lagen Mull geschüttet und für die spätere Verwendung aufgehoben. Die Pellets wurden in 200 ml STE resuspendiert und nochmals für 3 × 20 s im Mixer zerkleinert. Nach der erneuten Zentrifugation für 15 min bei 500 × g und 4°C wurden die Überstände durch die bereits benutzten Mulllagen geschüttet und die Pellets durch zwei neue Lagen Mull und eine Lage MiraCloth ausgedrückt, um die restliche Flüssigkeit zu erhalten. Die vereinigten Überstände (ca. 500 ml) wurden im Glas-Homogenisator durch 4-5 Hübe bei 100 rpm weiter zerkleinert und bei 4°C und 1500 × g für 15 min zentrifugiert. Die Zentrifugation wurde mit den Überständen mindestens zweimal wiederholt, bis nahezu kein Pellet mehr zu sehen war. Anschließend wurden die Mitochondrien aus dem Überstand bei 10.800 × g und 4°C für 20 min abzentrifugiert. Das Pellet wurde entweder für die Herstellung eines *in-vitro*-Transkriptionsextraktes verwendet oder in 120 ml STEM resuspendiert und mit zwei Hüben bei 100 rpm im Glas-Homogenisator gepottert. Es wurde in STEM gelöste DNase mit der Endkonzentration von 0,1 mg/ml zugegeben, die Proben auf vier 50-ml-Reaktionsgefäße verteilt und für 30 min bei 37°C im Wasserbad inkubiert. Nach der Inaktivierung der DNase I durch Zugabe von 7 ml 0,5 M EDTA pro Reaktionsgefäß und einer anschließenden Inkubation bei RT für 10 min wurden die Mitochondrien für 20 min bei 4°C und 15.000 × g abzentrifugiert. Die Pellets wurden in 80 ml STE+ aufgenommen, auf zwei 50-ml-Reaktionsaefäße verteilt und jeweils 0,8 ml 5 M NaCl zugegebenAnschließend wurde solange 0,5 M SDS zugegeben (ca. 0,5-1 ml), bis die Mitochondrien lysiert waren und die Lösung klar wurde. Die Lösung wurde danach mit 2,5 mg DNase-freier RNase A pro Reaktionsgefäß versetzt und im Wasserbad bei 37°C für 1 h inkubiert. Die Proben wurden vereinigt, auf sechs 50-ml-Reaktionsgefäße verteilt, einer Phenol-/Chloroform-Extraktion unterzogen, auf 16 Corex-Glas-Zentrifugenröhrchen verteilt und über Nacht bei -20°C ohne Zugabe von Natriumacetat gefällt. Nach dem Lufttrocknen wurden die Pellets jeweils in 500 µl ddH₂O aufgenommen, vereinigt, dialysiert und wiederum gefällt. Das Pellet wurde in 500 µl ddH2O aufgenommen, nach einer Restriktionsspaltung mit *Pvu*II gelelektrophoretisch analysiert und bei -20°C gelagert. Waren genügend Proben (5-8 Stück) aufgereinigt, wurden sie vereinigt, mit 50 µg/ml DNase-freier RNase A versetzt und für 1 h bei 37°C im Wasserbad inkubiert. Anschließend wurde eine Reinigung mittels CsCI-Gradienten-Zentrifugation durchgeführt. Nach der Dialyse wurde die DNA gefällt, in 100 µl ddH₂O aufgenommen und nach einer Restriktionsspaltung mit *Pvu*II gelelektrophoretisch sowie durch einen Southern Blot analysiert. Die DNA wurde bis zur weiteren Verwendung bei -20°C gelagert. Die aufgereinigten Mitochondrien aus humaner Placenta wurden in zwei Volumen MS-Puffer aufgenommen und anschließend für 10 min bei 4°C und 13.000 × g abzentrifugiert. Das Pellet wurde in einem Volumen ML-Puffer aufgenommen, mit 0,5% Tween 20 sowie 0,5% KCI versetzt und 10 Minuten auf Eis inkubiert. Das Lysat wurde im Glas-Homogenisator durch 10 Hübe bei 100 rpm suspendiert und bei 4°C und 13.000 × g für 45 min zentrifugiert. Der klare Überstand (S-13-Extrakt) wurde vorsichtig abgenommen, aliquotiert in flüssigem Stickstoff eingefroren und anschließend bei -80°C gelagert. Der Extrakt blieb mindestens ein Jahr lang aktiv.

### Beispiel 3: In-vitro-Transkription mitochondrialer Plasmide

### 3.1. Herstellung eines mitochondrialen in-vitro-Transkriptionsextraktes (modifiziert nach Fernandez-Silva, P. et al., Methods Enzymol., 1996, 164, 129-139)

Bei der *in-vitro*-Transkription erfolgt die Synthese der RNA anhand einer Template-DNA in einem zellfreien System. Da die Mitochondrien ein eigenes Transkriptionssystem besitzen, kann für die *in*-*vitro*-Transkription mitochondrialer Gene keines der etablierten Transkriptionssysteme genutzt werden. Durch die Herstellung eines Transkriptionsextraktes aus isolierten Mitochondrien ist es jedoch möglich, die Transkription mit mitochondrialen Promotoren zu nutzen.

### Benötigtes Material:

aufgereinigte Mitochondrien aus Placenta
MS-Puffer (*mitochondria suspension buffer*)
ML-Puffer (*mitochondria lysis buffer*)
Tween 20
5 M KCl

### Durchführung:

Die aufgereinigten Mitochondrien aus humaner Placenta wurden in zwei Volumen MS-Puffer aufgenommen und anschließend für 10 min bei 4°C und 13.000 × g abzentrifugiert. Das Pellet wurde in einem Volumen ML-Puffer aufgenommen, mit 0,5% Tween 20, sowie 0,5% KCI versetzt und 10 Minuten auf Eis inkubiert. Das Lysat wurde im Glas-Homogenisator durch 10 Hübe bei 100 rpm suspendiert und bei 4°C und 13.000 × g für 45 min zentrifugiert. Der klare Überstand (S-13-Extrakt) wurde vorsichtig abgenommen, aliquotiert in flüssigem Stickstoff eingefroren und anschließend bei -80°C gelagert. Der Extrakt blieb mindestens ein Jahr lang aktiv.

### 3.2. In-vitro-Transkription

Die Template-DNA muss vor dem Einsatz in der *in-vitro*-Transkription durch eine Restriktionsendonuklease stromabwärts des Promotors und des zu transkribierenden Fragmentes linearisiert werden. Geschieht dies nicht, erhält man aufgrund der Prozessivität der RNA-Polymerasen eine Vielzahl unterschiedlicher RNAs. Zum Schutz der entstehenden RNA-Moleküle vor eventuell vorhandenen RNasen ist die Zugabe eines RNase-Inhibitors sinnvoll.

### Benötigtes Material:

linearisierte Template-DNA
RiboLock™ RNase-Inhibitor
rNTP-Mix, 25 mM each
DEPC-H₂O
S-13-*in-vitro*-Transkriptionsextrakt
2 × Transkriptionspuffer
Transkriptions-Stop-Puffer

### Durchführung:

Zur *in*-*vitro*-Transkription wurde der folgende Ansatz mit einem Reaktionsvolumen von 100 µl auf Eis zusammenpipettiert:

| | |
|---|---|
| x µl | Template-DNA (2 µg) |
| 2,5 µl | RiboLock™ RNase-Inhibitor (100 U) |
| 4 µl | rNTP-Mix |
| ad 35 µl | DEPC-H₂O |
| 15 µl | S13-*in-vitro*-Transkriptionsextrakt |
| 50 µl | 2 × Transkriptionspuffer |

Der Ansatz wurde bei 30°C für 45 min inkubiert und anschließend wurde die Reaktion durch Zugabe von 200 µl Transkriptions-Stop-Puffer gestoppt.

### 3.3. Aufreinigung der RNA aus dem in-vitro-Transkriptionsextrakt

Um RNA aus dem Reaktionsansatz, der einen hohen Proteinanteil besitzt, isolieren zu können, wird der Ansatz einer Phenol-/Chloroform-Extraktion unterzogen. Durch den Einsatz von Phenol mit einem pH-Wert von 4,5-5 wird die RNA in der wässrigen Phase angereichert, während sich die DNA eher in der Interphase ansammelt, so dass die DNA-Kontamination im RNA-Eluat vermindert wird. Das Lösungsmittel Phenol stört nachfolgende enzymatische Reaktionen und muss deshalb sorgfältig durch mehrmaliges Ausschütteln mit Chloroform entfernt werden.

### Benötigtes Material:

Phenol pH 4,5-5
Phenol/Chloroform/Isoamylalkohol, 25:24:1 (v/v/v)
Chloroform/Isoamylalkohol, 24:1 (v/v)
Chloroform
DEPC-H₂O

### Durchführung:

Die RNA-Lösung wurde nacheinander mit einem Volumen Phenol, einem Volumen Phenol/Chloroform/Isoamylalkohol und einem Volumen Chloroform/Isoamylalkohol ausgeschüttelt. Dazwischen wurde jeweils bei RT bei 14.000 × g zentrifugiert, bis die Phasen vollständig getrennt waren und anschließend wurde die obere wässrige Phase, die die RNA enthielt, in ein neues Reaktionsgefäß überführt. Um sicherzustellen, dass kein Phenol mehr in der RNA-Lösung vorhanden ist, erfolgte die Chloroform/Isoamylalkohol-Extraktion bis zu fünf Mal (es durfte keine Interphase mehr zu sehen sein). Beim letzten Schritt wurde Chloroform statt Chloroform/Isoamylalkohol verwendet. Anschließend erfolgte eine Fällung der Nukleinsäuren mittels Ethanol. Das Pellet wurde in 25 µl DEPC-H₂O aufgenommen.

### Beispiel 4: Zellkultur

### 4.1. Kultivierung von Zellen

Die Kultivierung von Zellen wurde unter Einhaltung aller Sicherheitsmaßnahmen bei keimarmen Bedingungen durchgeführt. Das Zellwachstum wurde mithilfe eines Inversmikroskops mit Phasenkontrasteinrichtung überprüft. Grundsätzlich galten die in Tab. 2 angegebenen Richtwerte für die verwendeten Kulturgefäße.

**Tabelle 2: Richtwerte für die Kultivierung von Zellen**

| **Gefäß** | **Fläche (cm²)** | **Zelldichte beim Aussäen** | **Zelldichte bei Konfluenz** | **Wachstums-medium (ml)** | **PBS (ml)** | **Trypsin (ml)** |
|---|---|---|---|---|---|---|
| Flaschen | | | | | | |
| TC 25 | 25 | 0,7×10⁶ | 2,8×10⁶ | 5 | 2 | 1 |
| TC 80 | 75 | 2,0×10⁶ | 8,0×10⁶ | 15 | 6 | 2 |
| Schalen | | | | | | |
| 35 mm | 8 | 0,3×10⁶ | 1,2×10⁶ | 2,5 | 2 | 0,5 |
| 60 mm | 21 | 0,7×10⁶ | 3,2×10⁶ | 5 | 4 | 1 |
| 100 mm | 55 | 2,0×10⁶ | 8,0×10⁶ | 10 | 8 | 2 |
| 150 mm | 148 | 5,0×10⁶ | 2,1×10⁷ | 20 | 15 | 4 |

Um etwaige Kontaminationen mit Mykoplasmen zu detektieren, wurden regelmäßig

Kontrollen mit Hilfe des VenorGeM^{®} Mycoplasma Detection Kit nach Herstellerangaben durchgeführt. Die Zellen wurden routinemäßig bei 37°C und einer relativen Luftfeuchtigkeit von 95% im CO₂-begasten Brutschrank kultiviert und je nach Wachstumseigenschaften alle zwei bis vier Tage passagiert.

### 4.2. Transfektion eukaryontischer Zellen

### 4.2.1. Transfektion mit dem Transfektionsreagenz METAFECTENE^{®} PRO

METAFECTENE^{®} PRO ist ein polykationisches Transfektionsreagenz, das in Kombination mit einem neutralen Kolipid in liposomaler Form vorliegt. Der Eintritt von DNA in die Zellen findet statt, indem die zu transfizierende DNA zunächst in kompakte Strukturen komplexiert und endozytotisch in die Zellen aufgenommen wird. Innerhalb der Zelle wird die Freisetzung der Nukleinsäuren aus den Endosomen durch Puffereigenschaften der Komplexbildner bewerkstelligt, die zu einem Anstieg des osmotischen Druckes in dem Endosom führen, so dass die Membran aufbricht. Dieser Prozess wird durch die Eigenschaften der kationischen Lipide unterstützt, die durch die Ansäuerung durch Protonen die Endosomen destabilisieren (*Repulsive Membrane Acidolyis*). Da die Aufnahme der DNA in den Zellkern überwiegend während des Zusammenbruchs der Kernhülle (Mitose) erfolgt, sollte die Transfektion am besten mit stark proliferierenden Zellen durchgeführt werden.

### Benötigtes Material:

METAFECTENE^{®} PRO (Biotex, Martinsried, Deutschland)

### Durchführung:

Die Transfektionen erfolgten nach der mitgelieferten Anleitung (Stand: Juli 2005). Um eine mögliche Adsorption der Reagenzien an die Gefäßwände zu verhindern, wurden für die Transfektion ausschließlich Gefäße aus Polystyrol verwendet.

### 4.2.2. Transfektion mit dem Transfektionsreagenz FuGENE^{®} HD

Bei FuGENE® HD handelt es sich um ein nicht-liposomenbasiertes Transfektionsreagenz, das einen Komplex mit der zu transfizierenden DNA bildet und diese anschließend in die Zelle transportiert. Ebenso wie bei METAFECTENE^{®} PRO sollte eine Transfektion mit FuGENE^{®} HD nur an stark proliferierenden Zellen durchgeführt werden.

### Benötigtes Material:

FuGENE^{®} HD (Roche, Mannheim, Deutschland)

### Durchführung:

Die Transfektionen erfolgten nach der mitgelieferten Anleitung (Stand: Oktober 2005).

### 4.2.3. Transfektion mit dem Transfektionsreagenz MATra-A

Diese Transfektionsreagenz nutzt magnetische Nanopartikel (MagTag™), um die gewünschte DNA in die Zellen einzuschleusen. Im ersten Schritt wird die DNA an die Nanopartikel gebunden, welche dann durch magnetische Anziehung (mittels einer Magnetplatte, die unter die Kulturschale gestellt wird) in die Zellen eindringen und dort die DNA auf ihrer Oberfläche abgeben können. Diese Methode hat den Vorteil, dass die Zellmembran nicht durch chemische Reagenzien beeinflusst wird und sie auch bei Zellen gute Ergebnisse liefert, die mit auf Liposomen basierenden Transfektionsreagenzien nur schlecht zu transfizieren sind.

### Benötigtes Material:

MATra-A (IBA, Göttingen, Deutschland)
Magnetplatte

### Durchführung:

Die Transfektionen erfolgten nach der mitgelieferten Anleitung (Stand: Juli 2006).

### Beispiel 5: Selektion transfizierter Zellen durch Antibiotika

Für eine stabile Transfektion ist eine Selektion positiver Klone unabdingbar. Nur so wird eine Reinkultur an Zellen erzielt, die das gewünschte Konstrukt enthält.

### Benötigtes Material:

Selektionsantibiotikum

### Durchführung:

Die Selektion der transfizierten Zellen wurde über Antibiotika sichergestellt, für die auf den jeweiligen Konstrukten die entsprechenden Resistenzgene vorhanden waren, um ein Überleben der positiven Klone zu gewährleisten. Für jedes Antibiotikum wurde zunächst ermittelt, ab welcher Konzentration alle untransfizierten Zellen einer Kulturschale abstarben bzw. das Wachstum einstellten. Dazu wurden die verschiedenen Zelllinien in den entsprechenden Kulturmedien ausgesät und mit dem jeweiligen Antibiotikum in verschiedenen Konzentrationen inkubiert. Das selektive Medium wurde alle zwei Tage gewechselt und die Zellen über insgesamt acht Tage hinweg beobachtet. Als Negativkontrolle dienten Zellen, die ohne Antibiotikum in dem Standardmedium inkubiert wurden. Die benötigte Menge an Antibiotikum für die Selektion nach einer Transfektion wurde durch die Konzentration ermittelt, bei der nach vier bis fünf Tagen alle Zellen in der Kulturschale abgestorben waren. Mit der Selektion wurde zwischen 24 und 48 h nach der Transfektion begonnen.

### Beispiel 6: Selektion von mit mtDNA transfizierten ρ⁰-Zellen

Für die Selektion von mit mtDNA transfizierten ρ⁰-Zellen steht nur ein metabolischer Test zur Verfügung. Bei diesem Test wird die Abhängigkeit der ρ⁰-Zellen von Uridin und Pyruvat ausgenutzt, um die mit mtDNA repopulierten Zellen zu selektieren. Diese Zellen können im Gegensatz zu den ρ⁰-Zellen auch ohne Zugabe von Uridin und Pyruvat wachsen und bilden somit Klone auf der Kulturplatte, während die ρ⁰-Zellen nach einiger Zeit absterben.

### Benötigtes Material:

ρ⁰-Selektionsmedium

### Durchführung:

Die zu untersuchenden Zellen wurden enzymatisch von der Zellkulturschale abgelöst und auf eine 150-mm-Kulturschale mit Selektionsmedium neu ausgesät. Das Medium wurde ungefähr eine Woche lang alle zwei Tage und danach jeden vierten Tag gewechselt. Wenn nach drei bis vier Wochen keine Klone zu erkennen waren, wurde die Kulturschale entsorgt.

### Beispiel 7: Herstellung von dialysiertem FCS

Durch Dialyse können niedermolekulare Bestandteile des FCS schonend entfernt werden. Dies ist vor allem für die Verwendung des FCS im Selektionsmedium für ρ⁰-Zellen wichtig, da hierbei eventuell vorhandene Uridin- und Pyruvat-Moleküle stören.

### Benötigtes Material:

Dialyseschlauch, MWCO 3,5 kDa
Verschlussklammem
FCS-Dialyse-Puffer
Sterilfilter

### Durchführung:

Zunächst wurde das eine Ende des Dialyseschlauches doppelt umgefaltet und anschließend mit einer Verschlussklammer verschlossen. Danach wurde das FCS in den Dialyseschlauch eingefüllt und genauso wie am anderen Ende sorgfältig verschlossen. Dabei wurde darauf geachtet, dass sich keine Luftblasen im Inneren des Schlauches befanden. Der Schlauch wurde in einem 10-I-Kunststoffeimer mit 8 I FCS-Dialyse-Puffer so befestigt, dass er nicht mit dem Rührfisch in Berührung kam. Die Dialyse erfolgte bei 4°C, wobei der Dialyse-Puffer nach zwei und weiteren vier Stunden gewechselt wurde. Anschließend wurde die Dialyse noch über Nacht weitergeführt. Am nächsten Morgen wurde das FCS aus dem Schlauch entnommen, sterilfiltriert und aliquotiert bei -20°C gelagert.

### Beispiel 8: Färbung von Mitochondrien mit MitoTracker^{®}-Farbstoffen

Mit Hilfe der MitoTracker^{®}-Farbstoffe können selektiv Mitochondrien in lebenden Zellen gefärbt werden. Die rot fluoreszierenden MitoTracker^{®}-Farbstoffe sind Derivate entweder von Tetramethylrosamin oder X-Rosamin und enthalten eine thiolreaktive Chlormethyl-Einheit, die wahrscheinlich für ihre Assoziation mit den Mitochondrien verantwortlich ist.

### Benötigtes Material:

MitoTracker^{®} Red CMXRos bzw. MitoTracker^{®} Deep Red FM von Invitrogen, Karlsruhe, Deutschland

### Durchführung:

Zum Medium lebender Zellen wurde MitoTracker^{®} Red CMXRos oder MitoTracker^{®} Deep Red FM in einer 1:1.000-Verdünnung gegeben und 30 min bei 37°C im Brutschrank inkubiert. Anschließend erfolgte ein Medienwechsel und eine weitere Inkubation von 15 bis 120 Minuten im Brutschrank bei 37°C, bevor die Zellen am Mikroskop untersucht bzw. fixiert wurden.

### Beispiel 9: Induktion von Megamitochondrien

### 9.1. Induktion über Ansäuerung des Mediums mit Milchsäure

Die Ansäuerung des Kulturmediums mit Milchsäure stellt eine Möglichkeit dar, ein Anschwellen der Mitochondrien zu induzieren.

### Benötigtes Material:

2 M Milchsäure

### Durchführung:

Das Kulturmedium wurde bis zu einem pH-Wert von 6,4-6,5 mit 2 M Milchsäure versetzt und anschließend in einer Kulturschale im Brutschrank für einige Stunden bei 5% CO₂ und 37°C inkubiert. Danach wurde der pH-Wert nochmals überprüft und bei Bedarf nochmals mit 2 M Milchsäure eingestellt. Die Zellen wurden mit dem angesäuerten Kulturmedium für eine bis mehrere Stunden im Brutschrank inkubiert, bevor sie für die entsprechenden Versuche verwendet wurden.

### 9.2. Induktion über Zugabe von Valinomycin

Valinomycin ist ein K⁺-selektives Zyklodepsipeptid, welches als lonophor agiert und die oxidative Phosphorylierung entkoppelt. Dabei kommt es innerhalb weniger Stunden zu einem Anschwellen der Mitochondrien.

### Benötigtes Material:

Valinomycin-Stammlösung (10 mM in DMSO)

### Durchführung:

Die Induktion erfolgte durch Zugabe der Valinomycin-Stammlösung im Verhältnis von 1:1.000 zum Kulturmedium.

### 9.3. Induktion über Ansäuerung des Mediums mit Natriumacetat

### Benötigtes Material:

3 M Natriumacetat, pH 5,2

Durchführung:

Das Natriumacetat wurde in der gewünschten Endkonzentration zusammen mit dem Kulturmedium in einer 60-mm-Kulturschale für 20 min bei Raumtemperatur vorinkubiert und anschließend auf die ausgesäten Zellen gegeben.

### 9.4. Induktion über Ansäuerung des Mediums mit Essigsäure

### Benötigtes Material:

Essigsäure

### Durchführung:

Die Essigsäure wurde in der gewünschten Endkonzentration zusammen mit dem Kulturmedium in einer 60-mm-Kulturschale für 20 min bei Raumtemperatur vorinkubiert und anschließend auf die ausgesäten Zellen gegeben.

### Beispiel 10: Partikelbeschuss mit der Genkanone

Bei dem hier genutzten Gerät handelte es sich um das PDS-1000/He-System der Firma Bio-Rad.

### 10.1. Vorbereitung des Geräts

Zu Beginn jeder Nutzung muss das Gerät, sowie ein Teil der später genutzten Materialien vorbereitet werden. Dabei werden das gesamte Leitungssystem, sowie der Druckkolben mit Helium gespült. Außerdem werden die Macrocarrier für die spätere Nutzung vorbereitet.

### Benötigtes Material:

Helium-Druckflasche
Macrocarrier
Edelstahlhalter für Macrocarrier
*Rupture Disk* (entsprechend dem verwendeten Druck)
Stopping Screen

### Durchführung:

Zum Spülen des Systems mit Helium wurde der Druckkolben wie in der Anleitung beschrieben mit einer *Rupture Disk* verschlossen und eine Stopping Screen in den dafür vorgesehenen Halter (oberste Ebene) eingelegt. Nach dem Anlegen eines Kammervakuums wurde der Druckaufbau bis zum Reißen der Disk durchgeführt. Danach wurde die Kammer belüftet und der Druckkolben gleich wieder mit einer neuen *Rupture Disk* verschlossen. Die *Macrocarrier* wurden in die zugehörigen Edelstahlhalter eingelegt, wobei darauf zu achten war, dass die *Macrocarrier* genau in die dafür vorgesehen Rille eingepasst wurden.

### 10.2. Präparation der Goldpartikel

Vor der Beschichtung mit DNA müssen die Goldpartikel vorbereitet werden. Durch die Vorbehandlung soll sichergestellt werden, dass die Partikel steril und frei von sonstigen Verunreinigungen sind und dass sie möglichst nicht als Konglomerate vorliegen.

### Benötigtes Material:

Goldpartikel (0,6 µm, 1,0 µm oder 1,6 µm Durchmesser)
70% Ethanol p.a. (v/v)
50% Glycerin (v/v), steril
Steriles Wasser

### Durchführung:

Zunächst wurden 30 mg der Goldpartikel in einem 1,5-ml-Reaktionsgefäß abgewogen. Nun wurde 1 ml 70% Ethanol zugegeben, für 4-5 min auf dem Vortexer bei höchster Geschwindigkeit durchgemischt und für 15 min bei RT inkubiert. Die Goldpartikel wurden für 5 s abzentrifugiert (mittels der Short Spin-Taste) und der Überstand verworfen. Anschließend wurden die Goldpartikel in 1 ml sterilem Wasser für 1 min auf dem Vortexer gemischt und 3 min bei RT inkubiert. Daraufhin erfolgte eine Abzentrifugation der Goldpartikel und nach dem Verwerfen des Überstandes wurde dieser Waschschritt noch zweimal wiederholt. Zum Schluss wurden die Goldpartikel in 500 µl 50% Glycerin aufgenommen (Endkonzentration: 60 mg/ml) und für 3x 10 s durch Ultraschall vereinzelt. Die Lagerung erfolgte bei 4°C im Kühlschrank.

### 10.3. Beschichten der Goldpartikel mit DNA

Damit die DNA mit den Goldpartikeln in die Zelle eingebracht werden kann, muss die DNA erst auf die Partikel aufgebracht werden. Dies geschieht, indem die DNA im Beisein der Partikel durch CaCl₂ ausfällt. Dabei bindet die DNA auf der Oberfläche der Partikel und kann anschließend mit diesen präpariert werden. Durch die Zugabe von Spermidin kann dieser Vorgang noch verstärkt werden.

### Benötigtes Material:

Goldpartikel (60 mg/ml, aus 3.5.1.2)
DNA-Lösung (333 ng/µl)
2,5 M CaCl₂
0,1 M Spermidin
70% Ethanol p.a. (v/v)
100% Ethanol p.a.

### Durchführung:

Die vorbereiteten Goldpartikel aus 10.2. wurden nochmals durch Ultraschall vereinzelt und 60 µl in ein neues 1,5-ml-Reaktionsgefäß überführt. Um eine gleichmäßige Verteilung der DNA auf den Goldpartikeln sicherzustellen, erfolgte im Abstand von jeweils 10 s die Zugabe von 20 µl DNA-Lösung, 75 µl 2,5 M CaCl₂ und 30 µl 0,1 M Spermidin unter kontinuierlichem Mischen auf dem Vortexer (mittlere Geschwindigkeit). Nach weiteren 3 min auf dem Vortexer wurden die nun beschichteten Goldpartikel für 5-10 min bei RT stehen gelassen, damit sie sich am Boden des Reaktionsgefäßes absetzen konnten. Nach einer Zentrifugation für 2-3 s (keinesfalls länger! mittels Short-Spin-Taste) wurde der Überstand verworfen und die Goldpartikel unverzüglich mit einer Pipette in 180 µl 70% Ethanol resuspendiert. Nachdem die Goldpartikel sich für 5-10 min bei RT sedimentieren konnten erfolgte wiederum eine Zentrifugation für 2-3 s. Dieser Waschschritt wurde noch zweimal mit 100% Ethanol wiederholt, die Partikel in 60 µl 100% Ethanol aufgenommen und anschließend gleich zum Beschuss eukaryontischer Zellen eingesetzt.

### 10.4. Partikelbeschuss humaner Zellen

Humane Zelllinien sollten für den Beschuss mit Mikropartikeln in Kunststoff-Kulturschalen mit einem Durchmesser von 35 mm kultiviert werden. Da die maximale beschossene Fläche ungefähr der Größe einer 35-mm-Schale entspricht bringt der Einsatz größerer Schalen keine Vorteile.

### Benötigtes Material:

DNA-beschichtete Goldpartikel
Macrocarrier in den Edelstahlhaltern
*Rupture Disks* (entsprechend dem verwendeten Druck, meist 1800 psi)
Stopping Screens
humane Zellen auf 35-mm-Schale (30-70% Konfluenz, je nach Anwendung und Zelllinie)
Kulturmedium (evtl. mit Antibiotikum)

### Durchführung:

Die DNA-beschichteten Goldpartikel wurden nochmals sorgfältig mit einer Pipette resuspendiert und jeweils 10 µl auf die Mitte der vorbereiteten Macrocarrier getropft. Nach dem Trocknen (ca. 10 min) wurden ein Edelstahlhalter mit einem Macrocarrier sowie eine Stopping Screen laut Bedienungsanleitung in den dafür vorgesehenen Halter eingesetzt (Goldpartikel auf der Unterseite). Das Kulturmedium auf den Zellen wurde nahezu vollständig abgenommen und die Kulturschale unverzüglich in die Apparatur eingesetzt (zweite Ebene von unten). Nach dem Evakuieren (je nach Versuch, meist 25-27 in Hg) der Kammer erfolgte der Beschuss mit dem Druck entsprechend der eingesetzten Rupture Disk. Auf die Kulturschale wurde nach dem sofortigen Belüften der Kammer 2 ml Kulturmedium gegeben und anschließend die Schale in den Brutschrank gestellt. Dieses Vorgehen wurde mit den restlichen Kulturschalen (insg. sechs Stück) wiederholt. Der Erfolg des Partikelbeschusses wurde nach 24-48 h am Mikroskop kontrolliert.

### Beispiel 11: Konstruktion mitochondrialer Expressionsvektoren

Die Benennung der mitochondrialen Expressionsvektoren erfolgte nach folgendem Schema:
pMAGx-y (MAG: *Mitochondrial Artificial Genome*)

Dabei entspricht x der Gruppe (beginnend mit 11), die sich nach den in dem jeweiligen Vektor verwendeten Bestandteilen der mitochondrialen DNA sowie dem genutzten Promotor richtet (Tab. 3), und y der fortlaufenden Nummer innerhalb der jeweiligen Gruppe.

**Tabelle 3: Gruppeneinteilung der konstruierten mitochondrialen Expressionsvektoren**

| **Vektorgruppe** | **mitochondriale Bestandteile** | **genutzter Promotor** |
|---|---|---|
| 11 | D-Loop + TTS | L-Strang |
| 12 | D-Loop + TTS | H-Strang |
| 13 | D-Loop + TTS + L-Strang-Replikationsursprung | H-Strang |
| 14 | D-Loop + L-Strang-Replikationsursprung + 18S rRNA (Cam^{®}) | H-Strang |
| 15 | D-Loop, +18S rRNA(Cam^{®}) | H-Strang |
| 16 | D-Loop | H-Strang |
| 17 | D-Loop + L-Strang-Replikationsursprung | H-Strang |

### 11.1. pMAG11-1

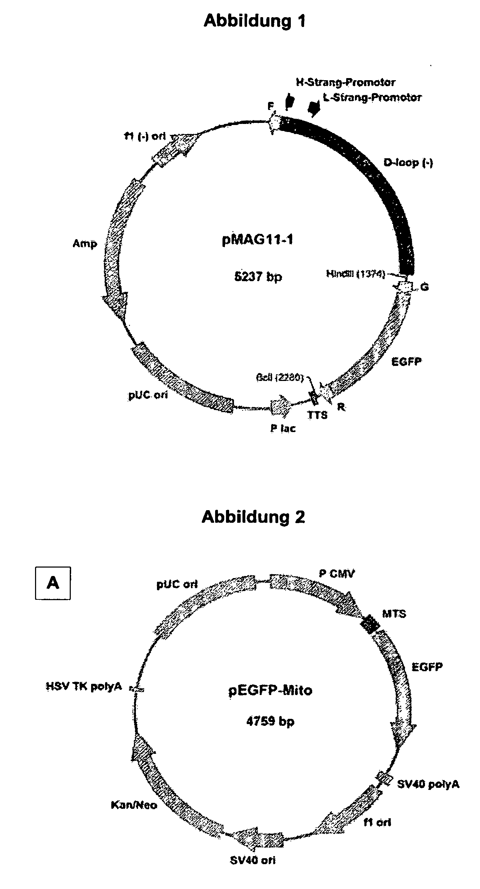

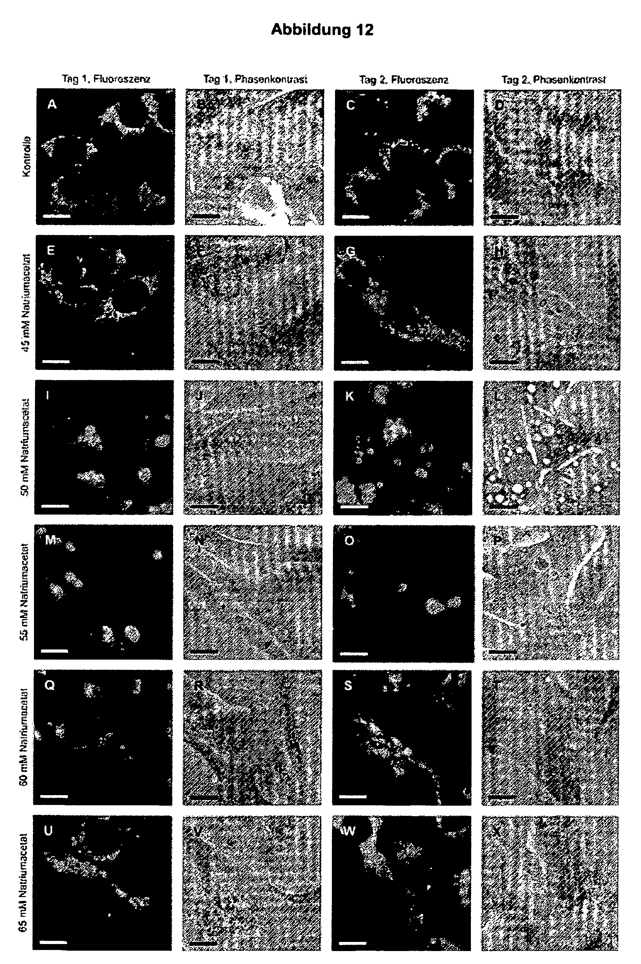

Bei diesem Vektor wurde EGFP als Reportergen eingebaut um einen einfachen und schnell nachzuvollziehenden Nachweis für eine gelungene Transfektion zu erhalten (Abb. 1). Die flankierenden tRNAs wurden so ausgewählt, dass sie in derselben Orientierung wie in der mitochondrialen DNA vorlagen. In diesem Fall wurden die tRNAs für Glycin und Arginin genutzt, die normalerweise das ND3-Gen flankieren, das eine Untereinheit der NADH-Ubiquinon-Oxidoreduktase codiert. Dabei war zu beachten, dass das Stop-Codon des ND3-Gens unvollständig ist d.h. dass es nur aus einem T, das durch die Polyadenylierung zu einem TAA vervollständigt wird, besteht (Ojala, D. et al., Nature, 1981, 290, 470-474).

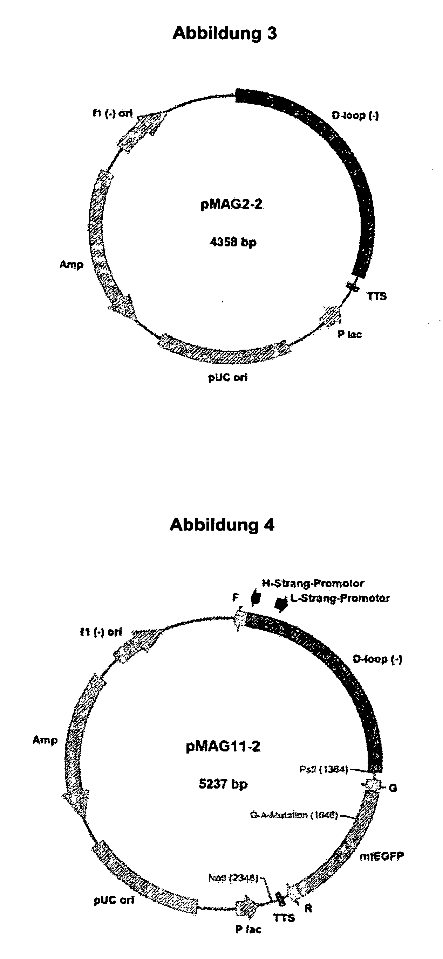

Die beiden tRNAs wurden jeweils aus vier Oligonukleotiden (GFP Link 1-4 bzw. GFP-Link 5-8) zusammengesetzt, die in einer Annealing-Reaktion aneinander binden konnten. An beiden Enden entstanden Überhänge mit einer Länge von vier Nukleotiden, um an das EGFP-Gen sowie den Vektor binden zu können. Das EGFP-Gen wurde aus dem Vektor pEGFP-Mito, der das Gen für EGFP trägt, an dessen C-Terminus das Signalpeptid der menschlichen Cytochrom-c-Oxidase Untereinheit 8 fusioniert wurde (Abb. 2A). mit den Primern EGFP-001-FOR (4) [5'-CTTTTGGTCTCAATGATGGTGAGCAAGGG-3'] und EGFP-716-REV (4) [5'-GTTAGTGGTCTCTATTTGTACAGCTCGTCC-3'] amplifiziert. Dabei wurden an den Enden Bsal-Schnittstellen angebracht, um zu den tRNAs kompatible Überhänge zu erhalten. Dieses Enzym spaltet die DNA nicht in ihrer Erkennungssequenz, sondern um ein Nukleotid versetzt in 3'-Richtung. Dabei entsteht ein vier Basen umfassender 5'-Überhang, der durch die Sequenz des verwendeten PCR-Primers definiert werden konnte. Anschließend wurden die tRNAs zusammen mit dem *B*sal-behandelten PCR-Produkt in den mit *Bcl*I und *Hind*III aufgeschnittenen Vektor pMAG2-2 (Abb. 3) ligiert, der wie der Vektor pMAG2-1 den vollständigen D-Loop der humanen mitochondrialen DNA enthält und zusätzlich die Transkriptions-Terminations-Sequenz (TTS) trägt. Der entstandene Vektor wurde sequenziert und erhielt den Namen pMAG11-1.

### 11.2. pMAG11-2

Bei dem Vektor pMAG11-2 wurde ein zusätzlicher Sicherheitsmechanismus eingebaut, um eine Expression des EGFP-Gens nach einer möglichen Integration in das Kerngenom zu verhindern. Bei der Mutagenese-PCR des Vektors pMAG11-1 wurden die Primer EGFP-156-FOR [5'-CAAGCTGCCCGTGCCCTGACCCACCCTCGTGACCAC-3'] und EGFP-191-REV [5'-GTGGTCACGAGGGTGGGTCAGGGCACGGGCAGCTTG-3'] eingesetzt, um das Tryptophan an der Stelle 58 (Trp58) des EGFP von TGG zu TGA zu verändern. Nach der Sequenzierung wurde das korrekte mitochondriale EGFP (mtEGFP) und einige umgebende Bereiche mit den Restriktionsendonukleasen *Pst*I und *Not*I ausgeschnitten und in den ebenso behandelten Vektor pMAG11-1 ligiert. Das neu entstandene Plasmid trug den Namen pMAG11-2 (Abb. 4).

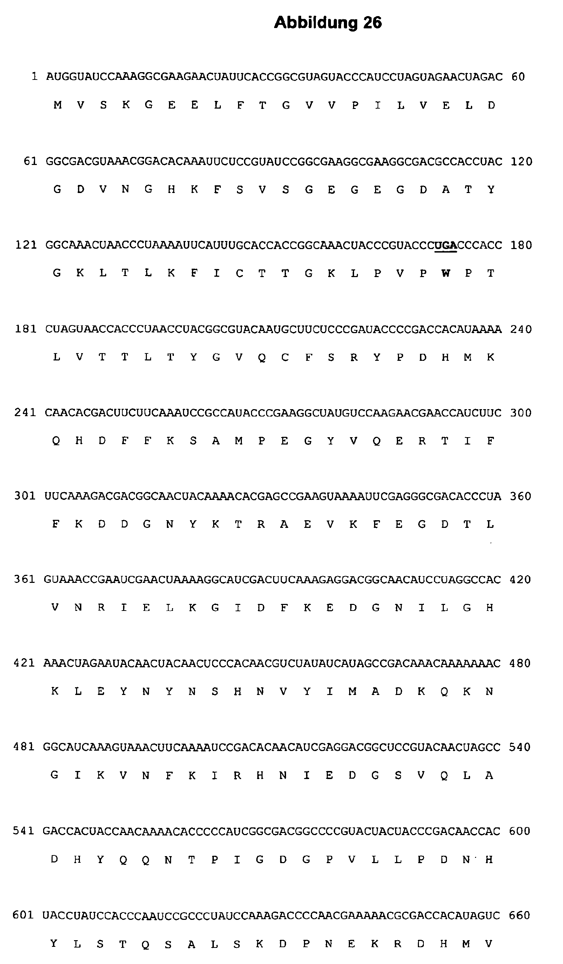

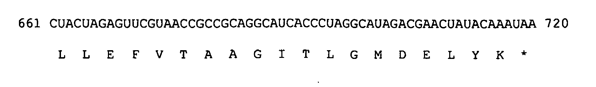

Alternativ zu dem hier aufgeführten mtEGFP kann auch ein vollständig an die Codon-Usage der Mitochondrien angepasstes GFP (mtoGFP) in diesem Vektor und den folgenden Vektoren verwendet werden. Es enthält auch die Veränderung des Trp58 und zusätzlich zahlreiche Veränderungen auf DNA-Ebene, die aber keine Auswirkungen auf restliche die Aminosäuresequenz bei einer Translation in Säuger-Mitochondrien haben. Durch diese Veränderungen wird jedoch die Translation in Mitochondrien optimiert und die Effizienz einer möglichen Translation (bis zum Stop-Codon an der Stelle 58) im Zytoplasma verringert (Abb. 26).

### 11.3. pMAG12-1

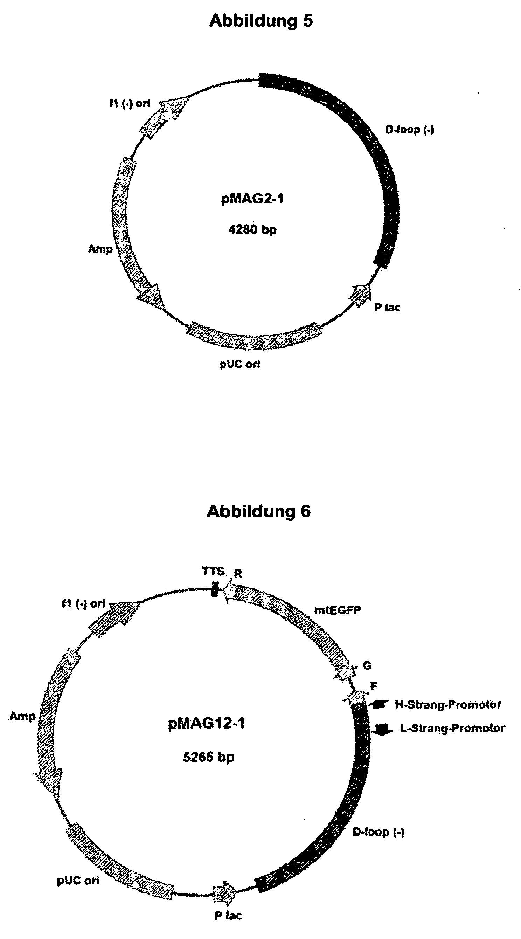

Um die Expression des mtEGFP zu optimieren, wurde das mtEGFP-Gen inklusive der angehängten tRNAs und der TTS in umgekehrter Orientierung auf die andere Seite des *D-Loops* kloniert. Dazu wurde das Plasmid pMAG2-1 (Abb. 5) mit *Xho*I und das Plasmid pMAG11-2 mit *Xho*I und *Nru*I geschnitten. Da die Enden nicht zueinander kompatibel waren, wurden alle freien DNA-Enden durch eine Behandlung mit dem Klenow-Fragment aufgefüllt. Nach der anschließenden Kontrolle der korrekten Orientierung der mtEGFP-Kassette wurde der Vektor sequenziert und erhielt den Namen pMAG12-1 (Abb. 6).

### 11.4. pMAG13-1

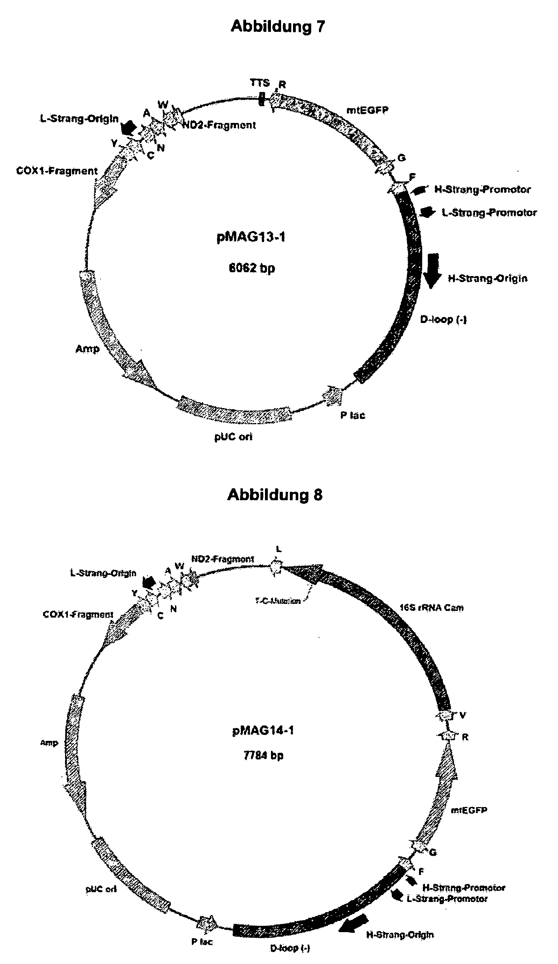

Der Vektor pMAG12-1 konnte zwar in Bakterien, nicht aber in den Mitochondrien repliziert werden, da er nur den Replikationsursprung für den H-Strang trug, der sich im *D-Loop*-Bereich befindet. Der Replikationsursprung für den L-Strang liegt in der mitochondrialen DNA jedoch in einem Bereich, der nicht im konstruierten Plasmid pMAG12-1 vorhanden war. Deshalb wurde ein 800 bp großer Bereich um den L-Strang-Replikationsursprung der mitochondrialen DNA in den Bereich des f1 (-) Origin des Vektors pMAG12-1 integriert. Der Bereich um den Replikationsursprung des L-Strangs wurde durch Amplifikation mit den Primern 05465-FOR und 06266-REV aus aufgereinigter Placenta-mtDNA gewonnen. Der Vektor pMAG12-1 wurde mit Hilfe der Restriktionsendonuklease *Dra*III linearisiert und die überstehenden 3'-Enden anschließend mit Klenow-Fragment entfernt. Nach der Ligation des PCR-Produktes in den Vektor wurde die Orientierung überprüft und der Vektor sequenziert. Der entstandene Vektor erhielt den Namen pMAG13-1 (Abb. 7).

### 11.5. pMAG14-1

Im Vektor pMAG14-1 (Abb. 8) enthielt eine Chloramphenicol-Resistenz (Cam^{R}) als zweites Markergen neben mtEGFP, das zur Ausübung eines Selektionsdrucks auf die Zellen genutzt werden konnte. Hierfür wurde das 16S rRNA-Gen zusammen mit den flankierenden tRNAs für Valin und Leucin aus einer Gesamt-DNA-Extraktion der Zelllinie HeLa 296-1, mit der gewünschten Chloramphenicol-Resistenz, mit Hilfe der Primer 01562-FOR [5'-GCGCTGCAGTAACATGGTAAGTGTACTGGAAAGTGCAC-3'] und 03351-REV [5'-AATCTCGAGATTAGAATGGGTACAATGAG-3'] amplifiziert und das PCR-Produkt anschließend in den Vektor ligiert. Nach einer Sequenzierung des gesamten Inserts wurde durch eine Restriktionsspaltung mit *Xho*I und *Bcl*I das 16S rRNA-Gen mit den tRNAs herausgeschnitten. Der Vektor pMAG13-1 wurde ebenfalls mit *Xho*I und *Bcl*I behandelt, so dass die TTS-Sequenz entfernt wurde. Das 16S rRNA-Gen konnte nun mit dem Vektorfragment zu dem neuen Vektor pMAG14-1 ligiert werden. Dabei wurde automatisch die vorher entfernte TTS-Sequenz wieder eingefügt, da sie sich in der Leucin-tRNA befindet, welche als flankierender Bereich der 16S rRNA mitkloniert wurde.

### 11.6. pMAG14-3

Der Vektor pMAG14-3 enthielt neben dem Replikationsursprung des L-Stranges, die Chloramphenicol-Resistenz und das Gen das für die T7-RNA-Polymerase codiert unter Kontrolle des mitochondrialen Promotors und das mtEGFP-Gen unter Kontrolle des T7-Promotors. Das mtEGFP-Gen wurde hinter der Leucin-tRNA am Ende des 16S rRNA-Gens in den Vektor integriert, wobei die tRNA, die die Transkriptions-Terminationsstelle enthält, den größten Teil der mitochondrialen Transkriptionsvorgänge stoppt.

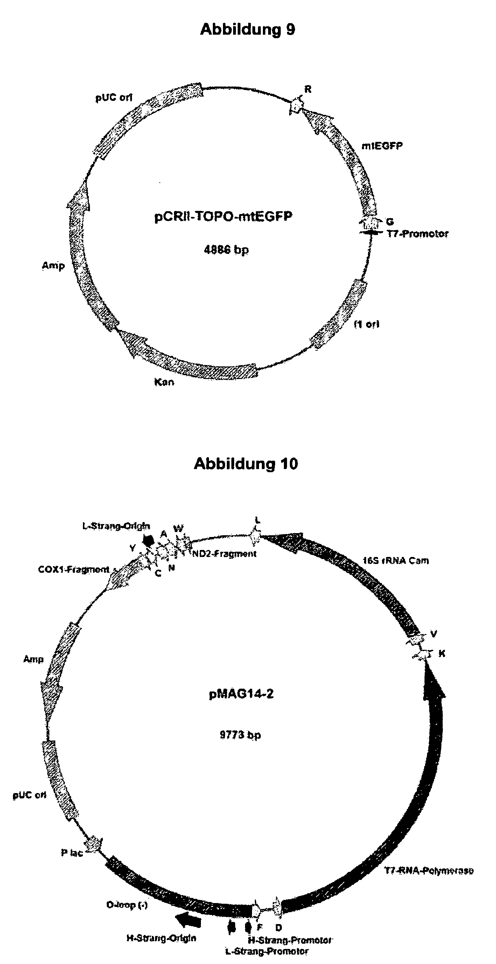

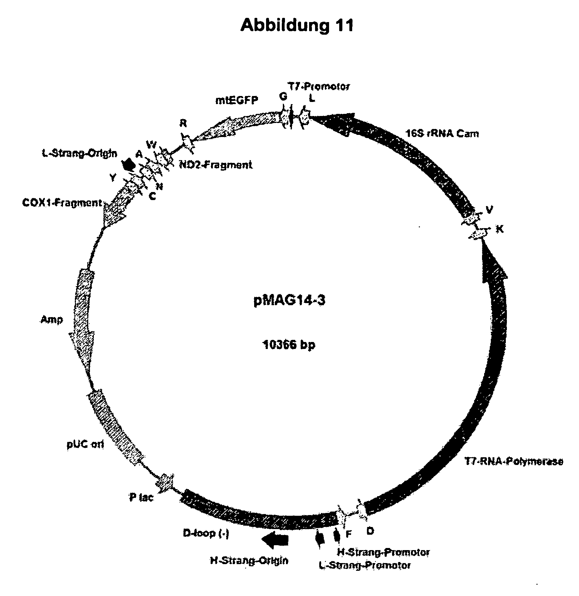

Zur Herstellung des Vektors pMAG14-3 wurde in einem ersten Schritt das mtEGFP aus dem Hilfsvektor pCRII-TOPO-mtEGFP (Abb. 9) mit den Restriktionsendonukleasen *Psi*I und *Xho*I herausgeschnitten und anschließend mit dem durch *Xho*I und *Nde*I aus dem Vektor pMAG14-2 gewonnenen und dephosphorylierten 16S rRNAGen über die *Xho*I-Übefiänge ligiert. Im zweiten Schritt wurde das entstandene DNA-Fragment aus 16S rRNA und mtEGFP in den mit *Psi*I und *Nde*I behandelten Vektor pMAG14-2 (Abb. 10) ligiert. Der entstandene Vektor erhielt die Bezeichnung pMAG14-3 (Abb. 11).

### Beispiel 12: Ergebnisse der Induktion von Megamitochondrien

### 12.1. Induktion mit Natriumacetat

Zwischen 2×10⁵ und 3×10⁵ Zellen der mit dem Plasmid pEGFP-Mito (Abb. 2A) transfizierten Zelllinie 143B.TK- wurden auf 35-mm-Glasbodenschalen ausgesät und in Kulturmedium mit unterschiedlichen Konzentrationen (45, 50, 55, 60 und 65 mM) Natriumacetat kultiviert. Am Tag 1 und Tag 2 nach der Zugabe des angesäuerten Mediums wurden die Zellen am Mikroskop auf die Bildung von vergrößerten Mitochondrien untersucht. Während die Kontrollzellen in Natriumacetat-freiem Medium nach dem ersten Tag ein Netzwerk aus dünnen schlauchförmigen Mitochondrien enthielten (Abb. 12, A+B), zeigten die Zellen mit 45 mM Natriumacetat erste Ansätze von verdickten Mitochondrien (Abb. 12, E+F). Bei 50 und 55 mM waren fast kugelförmige Mitochondrien mit einem Durchmesser von bis zu 4 µm erkennbar (Abb. 12, I+J und M+N), während in denselben Zellen teilweise auch noch ein schlauchförmiges Netzwerk zu sehen war. Bei den weiter erhöhten Konzentrationen von 60 und 65 mM Natriumacetat nahm die Größe der Mitochondrien wieder ab und auch ihre Form veränderte sich zu teils tropfenförmigen Verdickungen des Netzwerks (Abb. 12, Q+R und U+V).

Am zweiten Tag war das Kulturmedium der Kontrollzellen leicht orangefarbig und auch die Mitochondrien bildeten kein Netzwerk mehr, sondern lagen fast punktförmig vor (Abb. 12, C+D). Bei 45, 55, 60 und 65mM zeigten sich nahezu keine Veränderungen zum Vortag (Abb. 12, G+H, O+P, S+T und W+X), während die Mitochondrien der Zellen in 50 mM Natriumacetat nun nur noch als Kugeln mit bis zu 6 µm Größe vorlagen (Abb. 12, K+L). In weiteren Versuchsreihen stellte sich heraus, dass die optimale Konzentration bei jeder neuen Kulturmedium- und FCS-Charge neu zu bestimmen war; sie lag aber meist zwischen 50 und 60 mM Natriumacetat.

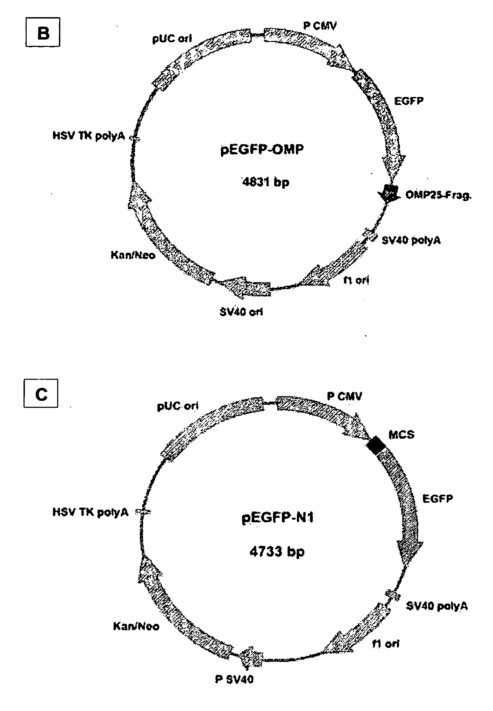

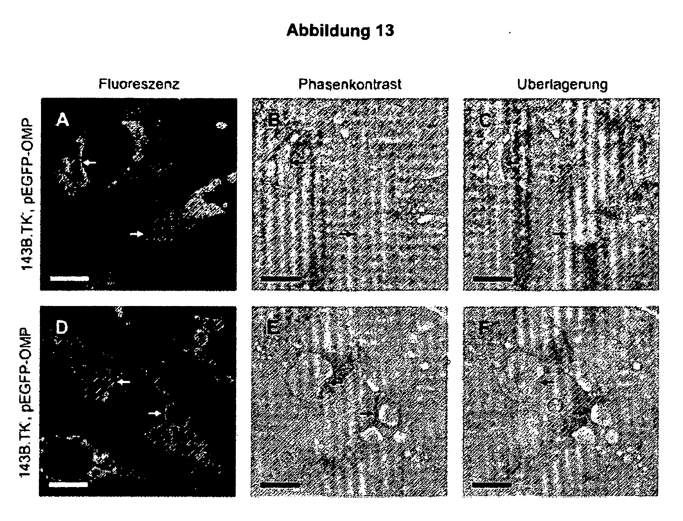

Bei einigen Versuchen konnte ein Anschwellen der Mitochondrien bis fast auf die Größe des Zellkerns erzielt werden. In Abb. 13 sind solche riesigen Megamitochondrien zu sehen (Pfeile bei A und D). Bei der hier verwendeten Zelllinie handelte es sich wiederum um die Zelllinie 143B.TK-, die stabil mit pEGFP-OMP (Abb. 2B) transfiziert wurde und bei der deshalb die äußere Mitochondrienmembran angefärbt war.

### 12.2. Induktion mit Essigsäure

Für die Induktion der Megamitochondrien mit Essigsäure wurde das Kulturmedium mit unterschiedlichen Konzentrationen (25-40 mM) Essigsäure versetzt und anschließend in ihm 2×10⁵ bis 3×10⁵ Zellen der mit pEGFP-Mito (Abb. 2A) transfizierten Zelllinie 143B.TK- in 30 mm Glasbodenschalen weiterkultiviert. Nach einem und einem weiteren Tag erfolgte eine Untersuchung am Mikroskop.

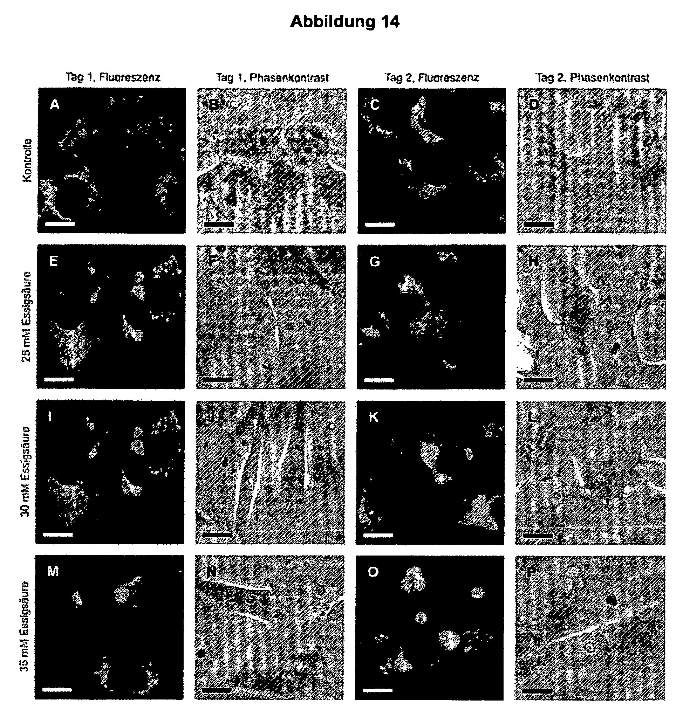

Die Zellen in 40 mM Essigsäure waren schon am ersten Tag abgestorben, während die anderen Konzentrationen keine Auswirkungen auf die Lebensfähigkeit der Zelllinie zeigten. Wie erwartet zeigten die Zellen der Kontrolle ein fadenförmiges Mitochondriennetzwerk (Abb. 14, A+B), während die Zellen bei den beiden niedrigen Essigsäurekonzentrationen bis 1 µm durchmessende Verdickungen des Netzwerks aufwiesen (Abb. 14, F+G und J+K). Bei der 35 mM Essigsäure konnten zusätzlich auch einzelne Mitochondrien mit einem Durchmesser von bis zu 5 µm entdeckt werden (Abb. 14, M+N).

Am Tag 2 zeigten sich bei der Kontrolle und der Zelllinie in 25 mM Essigsäure keine nennenswerten Veränderungen (Abb. 14, C+D und G+H), bei den Mitochondrien der Zellen in 30 mM Essigsäure wurden vereinzelt Durchmesser von 4 µm gemessen (Abb. 14, K+L). Die Zellen mit der höchsten Konzentration Essigsäure konnten gegenüber dem Vortag noch einmal vergrößerte Riesenmitochondrien vorweisen, die zum Teil 7 µm durchmaßen. Gleichzeitig war in einigen Zellen immer noch ein schlauchförmiges Netzwerk zu erkennen (Abb. 14, O+P).

### Beispiel 13: Ergebnisse der Methoden zur Transfektion von Mitochondrien mit mitochondrialen Expressionsplasmiden

### 13.1. Partikelbeschuss mit der Genkanone

### 13.1.2. Bestimmung der Durchführungsbestimmungen

Um die Variablen einzugrenzen, wurde ein Vergleich zu der zytoplasmatischen Transfektion der später verwendeten Zelllinie 143B.TK- angestellt. In ersten Vorversuchen wurde das Streuungsverhalten der Partikel in Abhängigkeit von der verwendeten Einschubebene (A-D) untersucht. Dabei stellte sich heraus, dass bei Nutzung der untersten Ebene (D) viele Goldpartikel über den Rand der verwendeten 35-mm-Kulturschalen hinaus verteilt wurden. Ein Einsetzen des Kulturschalenträgers auf der obersten Ebene (A) direkt unterhalb des Macrocarrier-Halters mündete in einem Trefferbild, bei dem nur eine rund 1 cm durchmessende kreisförmige Region in der Mitte der Schale von den Goldpartikeln getroffen wurde. In dieser Region lagen die Partikel so dicht aneinander, dass dort - entweder durch den plötzlich einwirkenden Heliumstrahl oder direkt durch den Aufschlag der Partikel - keine Zellen mehr zu finden waren. Bei der nächsten darunterliegenden Ebene (B) konnte ein ähnlicher, jedoch abgeschwächter Effekt beobachtet werden: Die Goldpartikel konnten nur in einem ungefähr zwei cm durchmessenden Bereich gefunden werden und auch hier zeigte sich, dass ein großer Teil der Zellen innerhalb dieser Region abgelöst bzw. zerstört wurden. Bei der Verwendung der zweituntersten Ebene (C) war der Anteil an abgelösten Zellen viel niedriger und die Goldpartikel zeigten ein homogeneres Verteilungsmuster über fast die gesamte Fläche der Kulturschale. Jedoch konnte auch in der Schalenmitte weiterhin ein kleiner Bereich ausgemacht werden, bei dem die Goldpartikel in einer relativ hohen Konzentration zu finden waren. Da hier die besten Ergebnisse bezüglich der Partikelverteilung und der Überlebensrate der Zellen erzielt werden konnten, wurden alle folgenden Versuche mit Kulturschalen auf dieser Ebene durchgeführt.

Im nächsten Schritt erfolgte die Optimierung der Kombination aus angelegtem Unterdruck (in *in Hg*, *inches of mercury*) und der verwendeten Rupture Disk-Drücke (in *psi*, *pounds per square inch*). Dafür wurden je zwei 35 mm-Kulturschalen mit Zellen (ca. 40-60% Konfluenz) mit je 600 µg der 0,6 µm durchmessenden Goldpartikel beschossen, die vorher mit 600 ng des Plasmids pEGFP-N1 (Abb. 2C) beschichtet wurden. Zum Vergleich der Transfektionsrate mit herkömmlichen Methoden erfolgte zusätzlich eine Transfektion einer Kulturschale mit dem Reagenz FuGENE^{®} HD (Fa. Roche, Mannheim, Deutschland). Nachdem alle Kombinationen aus 15, 20, 25 und 27 in Hg Unterdruck und den *Rupture Disks* für 1350, 1550, 1800 und 2000 psi angewendet wurden, erfolgte am nächsten Tag die Untersuchung am Fluoreszenzmikroskop. Es wurden jeweils mehrere Fluoreszenz- und Phasenkontrastaufnahmen angefertigt und anschließend mit ihnen die transfizierten sowie die Gesamtzahl der Zellen durch Auszählen ermittelt. Die Ergebnisse dieser Zählung und die daraus errechneten Transfektionsraten sind in der nachfolgenden Tabelle 4 aufgeführt:

**Tabelle 4: Bestimmung der Transfektionsrate unter verschiedenen Reaktionsbedingungen**

| **Versuchsbedingungen (*Rupture Disk*/*Unterdruck*)** | **Gesamtzahl der Zellen** | **Anzahl transfizierter Zellen** | **Transfectionsrate** |
|---|---|---|---|
| 1350 psi / 15 in Hg | 13534 | 3 | 0,022% |
| 1550 psi / 15 in Hg | 13038 | 4 | 0,031% |
| 1800 psi / 15 in Hg | 13700 | 5 | 0,036% |
| 2000 psi / 15 in Hg | 13886 | 7 | 0,050% |
| 1350 psi / 20 in Hg | 19014 | 12 | 0,063% |
| 1550 psi / 20 in Hg | 21808 | 16 | 0,073% |
| 1800 psi / 20 in Hg | 18730 | 16 | 0,085% |
| 2000 psi / 20 in Hg | 18828 | 9 | 0,048% |
| 1350 psi / 25 in Hg | 15492 | 23 | 0,148% |
| 1550 psi / 25 in Hg | 14560 | 65 | 0,446% |
| 1800 psi / 25 in Kg | 20572 | 95 | 0,462% |
| 2000 psi / 25 in Hg | 17810 | 61 | 0,343% |
| 1350 psi / 27 in Hg | 2888 | 85 | 2,94% |
| 1550 psi / 27 in Hg | 2179 | 140 | 6,42% |
| 1800 psi / 27 in Hg | 3846 | 310 | 8,06% |
| 2000 psi / 27 in Hg | 3348 | 127 | 3,79% |
| FuGENE^{®} HD | 20102 | 4372 | 24,7% |

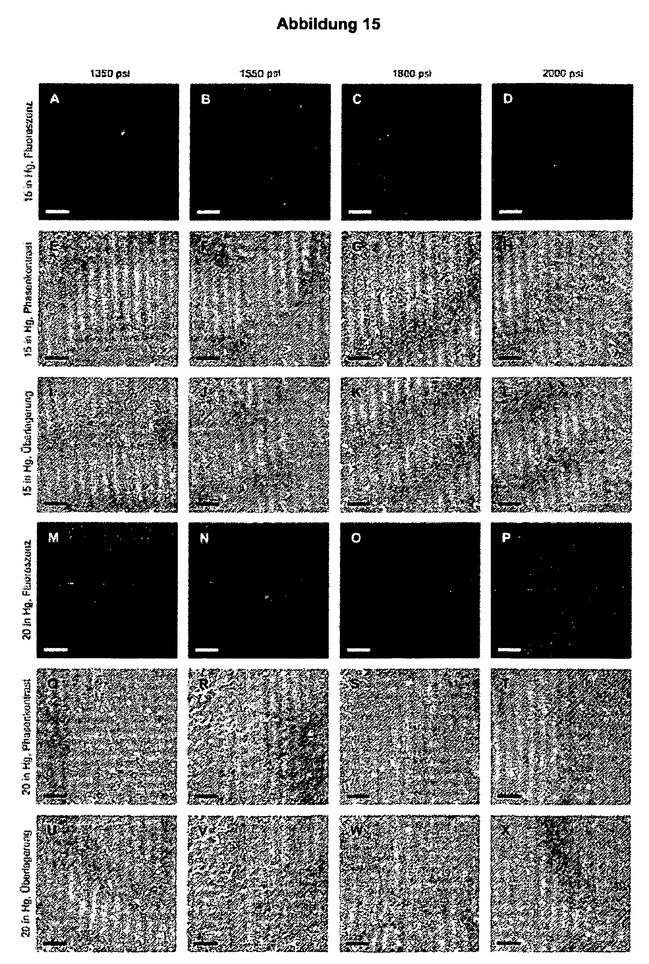

Bei dem Kammervakuum von 15 in Hg konnten nur vereinzelt transfizierte Zellen gefunden werden (Abb. 15, A-D). Die Transfektionsraten erreichten dementsprechend nur sehr niedrige Werte, die sich mit steigendem Schussdruck von 0,022 auf 0,050% erhöhten. Die Steigerung des Kammervakuums auf 20 in Hg brachte nur wenig Besserung: Die Transfektionsraten schwankten zwischen 0,048 und 0,085%, was auch auf den entsprechenden Aufnahmen (Abb. 15, M-P) anhand weniger Transfektanten zu sehen war. Erneut zeigte sich, dass sich die Transfektionsrate mit steigendem Druck erhöhte, jedoch bei Verwendung der stärksten *Rupture Disks* (2000 psi) auf den niedrigsten Wert absank.

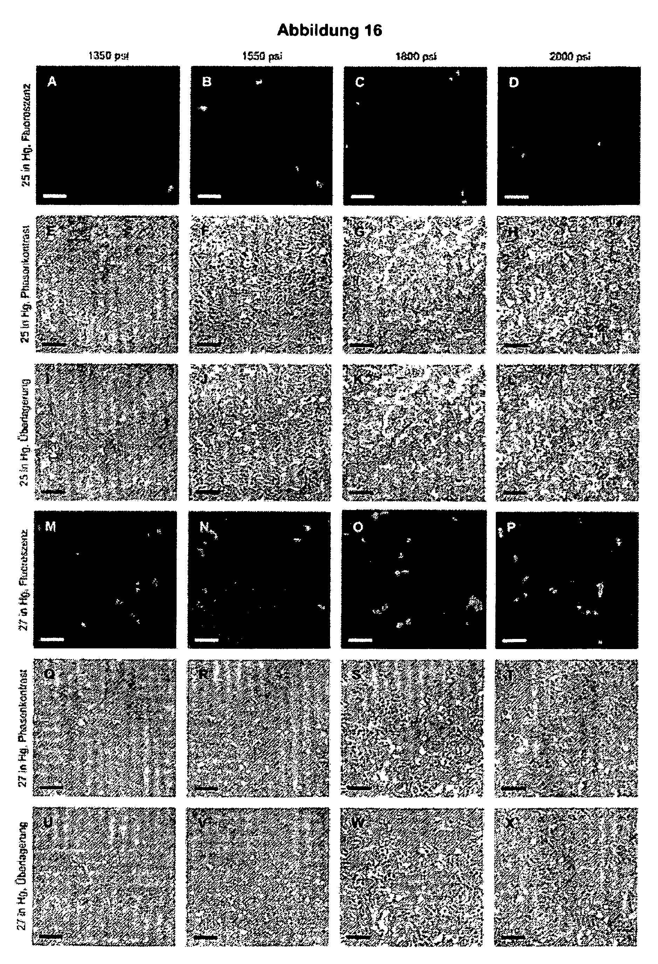

Bei 25 in Hg Kammervakuum waren auf den Fluoreszenzaufnahmen vermehrt grün leuchtende Zellen zu erkennen (Abb. 16, A-D) und auch die Transfektionsrate stieg dementsprechend auf bis zu 0,462% bei 1800 psi Schussdruck an. Durch die Verwendung des höchsten Kammervakuums von 27 in Hg konnte die Transfektionsrate nochmals um ungefähr Faktor 20 erhöht werden: Es wurden Werte zwischen 2,94% bei Verwendung von *Rupture Disks* für 1350 psi und 8,06% bei 1800 psi*-Rupture Disks* erzielt. Dies konnte auch durch die Betrachtung der Fluoreszenzaufnahmen bestätigt werden (Abb. 16, M-P).

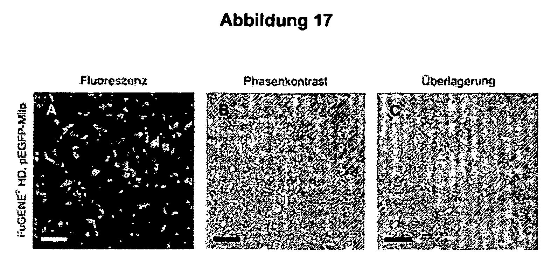

Im Vergleich dazu lieferte der Einsatz des Transfektionsreagenz FuGENE^{®} HD eine nochmals 3-fach erhöhte Transfektionsrate von 24,7% (Abb. 17).

Aufgrund dieser Ergebnisse wurden die weiteren Transfektionsversuche an der Genkanone mit den folgenden Parametern durchgeführt:
- Nutzung der zweiten Ebene von unten,
- Kammervakuum von 27 in Hg,
- Verwendung von *Rupture Disks* für 1800 psi.

### 13.1.3. Induktion von Megamitochondrien

Da Mitochondrien nur einen Durchmesser von 0,5-1 µm aufweisen, ist es nahezu unmöglich, sie in lebenden Zellen mit Goldpartikeln von 0,6 µm so zu treffen, dass die DNA auf den Goldpartikeln die Mitochondrienmatrix erreicht.

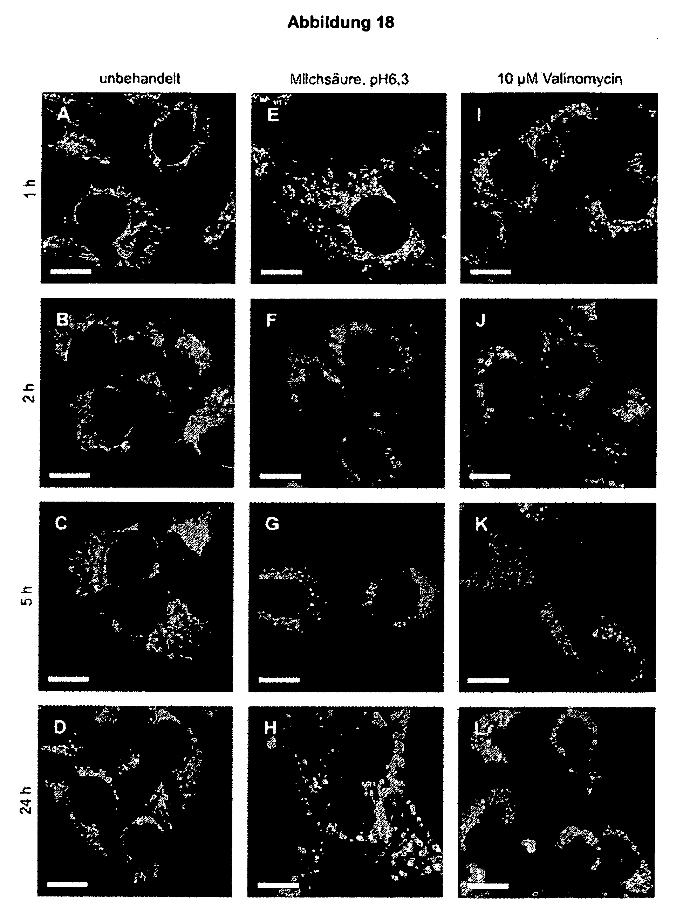

Zu Beginn wurde ausgetestet, welche Methode am besten geeignet ist, um Megamitochondrien vor den Transfektionsversuchen mit der Genkanone zu induzieren. Dazu wurde die stabile Zelllinie 143B.TK⁻+pEGFP-Mito, die grüne Mitochondrien besitzt, über einen Tag zum einen in mit Milchsäure auf einen pH-Wert von 6,3 eingestelltem Kulturmedium und zum anderen mit Kulturmedium, das 10 µm Valinomycin enthielt, kultiviert. Als Kontrolle wurde dieselbe Zelllinie in unverändertem Kulturmedium mitgeführt. Nach einer, zwei, fünf und 24 Stunden erfolgte eine Untersuchung am Fluoreszenzmikroskop. Die Mitochondrien in der Kontrollzelllinie zeigten in den ersten fünf Stunden keine Morphologieänderungen - sie bildeten ein Netzwerk ohne nennenswerte Verdickungen (Abb. 18, A-C). Nach einem Tag jedoch war das Kulturmedium leicht orange gefärbt und einige der Mitochondrien waren kugelförmig angeschwollen bzw. verdickt (Abb. 18, D). Bei der mit Milchsäure behandelten Zellline war nach einer Stunde das gesamte mitochondriale Netzwerk aufgelöst und es konnten nur noch kugelförmige Mitochondrien erkannt werden, die zum Teil schon eine Vergrößerung zeigten (Abb. 18, E). In den folgenden Stunden und am nächsten Tag konnte eine weitere Größenzunahme festgestellt werden, so dass die Megamitochondrien sehr deutlich zu sehen waren (Abb. 18, F-H). Die Zugabe von Valinomycin induzierte ebenfalls schon nach einer Stunde kugelförmige Mitochondrien, die allerdings etwas größer als im Milchsäuremedium waren, was auch nach zwei Stunden der Fall war (Abb. 18, I+J). Nach fünf bzw. 24 Stunden konnten nahezu keine Unterschiede in der Mitochondriengröße zwischen den Versuchsreihen festgestellt werden (Abb. 18, K+L).

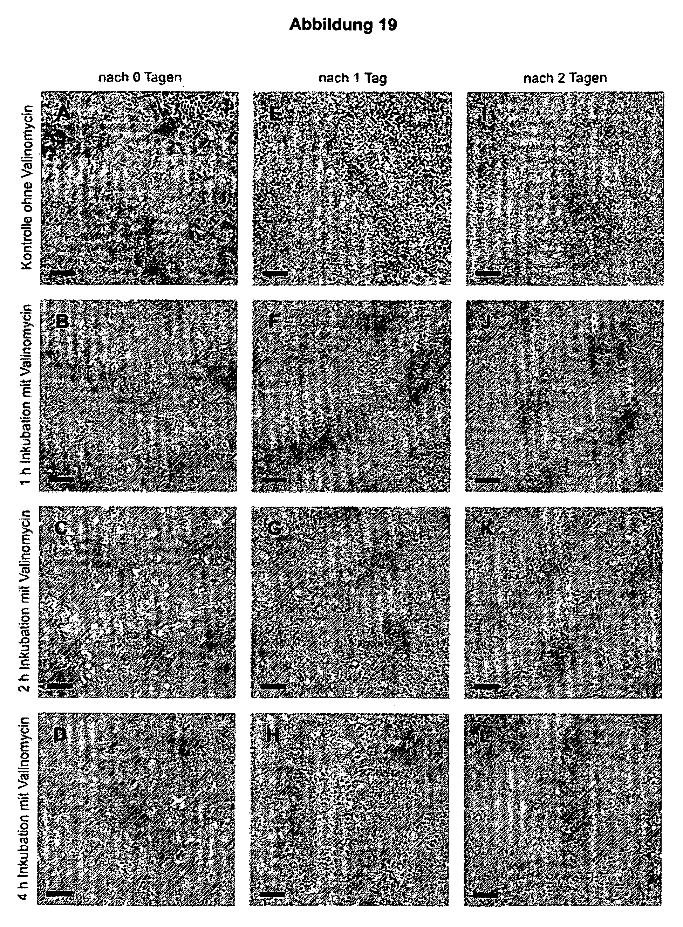

Bei der Beobachtung der mit Valinomycin behandelten Zelllinie fiel auf, dass sich die Zellen nach 24 Stunden nahezu nicht vermehrt hatten. Da dies zu Problemen bei der folgenden Transfektion führen könnte, wurde eine Versuchsreihe durchgeführt, bei der die Zelllinie 143B.TK⁻ für eine, zwei und vier Stunden in Kulturmedium mit 10 µM Valinomycin inkubiert wurde. Als Kontrolle diente dieselbe Zelllinie in Valinomycin-freiem Medium. Nach der Inkubation wurden die Zellen dreimal für 10 Minuten in normalem Kulturmedium gewaschen, um das Valinomycin zu entfernen. Im Anschluss an die Waschschritte erfolgten Aufnahmen am Mikroskop, um die Ausgangsdichte der Zellen zu dokumentieren (Abb. 19, A-D). An den zwei folgenden Tagen wurden erneut Aufnahmen angefertigt. Dabei zeigte sich, dass sich unabhängig von der Inkubationsdauer alle mit Valinomycin behandelten Zellen im Gegensatz zu den Kontrollzellen nahezu nicht mehr teilten (Abb. 19, E-H). Die Aufnahmen vom zweiten Tag nach der Inkubation zeigten sogar eine Abnahme der Zelldichte, was den Schluss zuließ, dass einige Zellen nach der Valinomycinbehandlung abstarben (Abb. 19, I-L). Die schnelle Induktion von Megamitochondrien durch Valinomycin wäre wünschenswert, aber aufgrund dieser Beobachtungen wurde von der Induktion von Megamitochondrien durch die Zugabe von Valinomycin zum Kulturmedium zunächst abgesehen, da nicht bekannt war, ob dadurch das Transfektionsergebnis beeinflusst werden könnte.

In einer weiteren Versuchsreihe sollte untersucht werden, ob die Dauer der Inkubation in angesäuertem Medium einen Einfluss auf die anschließende Transfektion mit der Genkanone hat. Dabei zeigte sich, dass eine Inkubation für mehr als zwei Stunden zu einem verstärkten Ablösen der Zellen beim Beschuss mit Goldpartikeln führte. Aus diesem Grund wurden die Zellen bei den folgenden Versuchen ungefähr 60 bis 120 Minuten vor Versuchsbeginn mit Milchsäure-Medium behandelt.

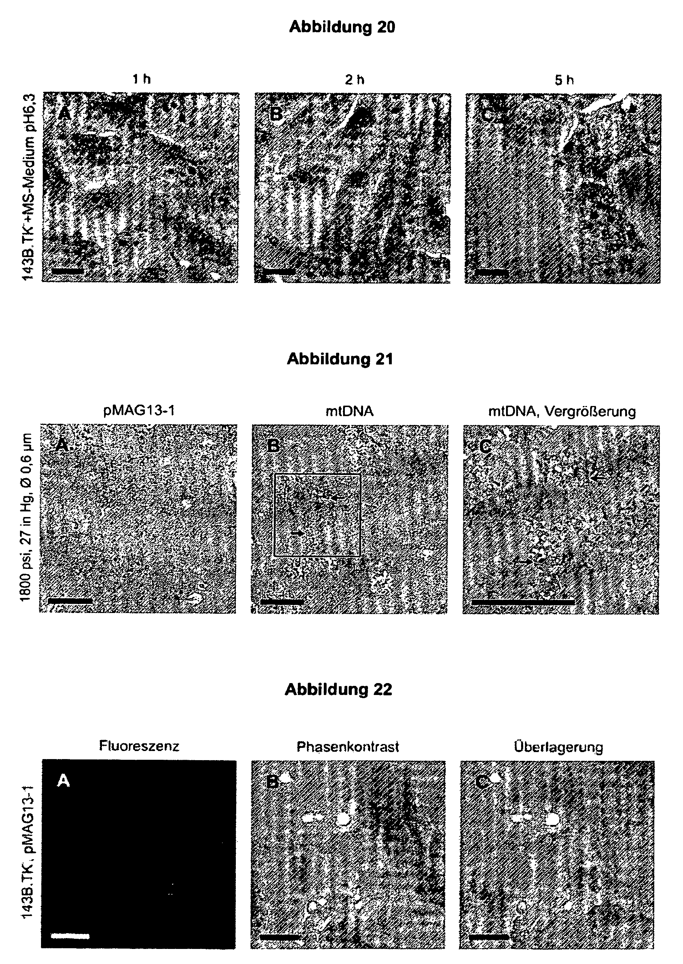

Nach dieser Zeit waren die vergrößerten Mitochondrien bereits im Phasenkontrast sichtbar (Abb. 20, A+B). Nach fünf Stunden Inkubation waren diese - wie nach den Ergebnissen der vorigen Versuchsreihe erwartet - noch besser sichtbar (Abb. 20, C).

### 13.1.4. Transfektion mit mtDNA

Zu Beginn wurde pro Transfektionsansatz 1 µg einer Cäsiumchloridgradienten-gereinigten mtDNA mit 0,5 µg Goldpartikeln mit einem Durchmesser von 0,6 µm verwendet. Schon beim Beschichten der Goldpartikel zeigte sich eine starke Aggregatbildung, die nach dem Beschuss der ρ⁰-Zelllinie 143B.TK- K7 auch lichtmikroskopisch auf der Kulturplatte nachgewiesen werden konnte. Im Vergleich zu mit dem Plasmid pMAG13-1 beschichteten Goldpartikeln (Abb. 21, A) war bei der Verwendung von mtDNA deutlich eine Bildung großer Aggregate zu erkennen (Abb. 21, Pfeile bei B+C). Die Verteilung der Goldpartikel beschränkte sich größtenteils auf einen 1-1,5 cm durchmessenden Bereich in der Mitte der Kulturschale, in dem sich auch die Zellen sehr stark ablösten. Um diese vermutlich DNA-abhängige Aggregatbildung zu reduzieren, wurde die Menge der mtDNA auf 0,5 µg pro Transfektionsansatz verringert, wodurch tatsächlich eine leichte Besserung auftrat. Zusätzlich wurden auch Goldpartikel mit 1,0 bzw. 1,6 µm Durchmesser ausgetestet. Auch hier konnte eine weitere leichte Verringerung der Aggregation festgestellt werden. Aufgrund dieser Resultate erfolgten je 18 Transfektionen mit 0,5 µg mtDNA und 0,5 µg Goldpartikeln mit den drei aufgeführten Durchmessern. Die Zellen wurden nach einer eintägigen Kultivierung in ρ⁰-Medium auf 150-mm-Kulturschalen umgesetzt und in ρ⁰-Selektionsmedium weiterkultiviert. Nach jeweils drei bis vier Wochen konnten jedoch keine lebenden Klone gefunden werden.

### 13.1.5. Transfektion mit mitochondrialen Expressionsvektoren

Vor Beginn der jeweiligen Versuchsreihen wurden je fünf 35-mm-Kulturschalen mit dem verwendeten Plasmid und dem Transfektionsreagenz FuGENE^{®} HD transfiziert, um eine Kontamination der DNA-Präparation mit anderen EGFP-exprimierenden Vektoren ausschließen zu können. Wenn am nächsten Tag keine grün fluoreszierenden Zellen gefunden wurden, konnte die DNA-Präparation für die Transfektionsversuche mit der Genkanone eingesetzt werden.

Pro Transfektionsansatz wurden 1 µg des jeweiligen Plasmids und 0,5 µg Goldpartikel mit dem Durchmesser von 0,6 µm verwendet. Damit die Zellen nach 1,5-2 Tagen noch gut beobachtet werden konnten, wurden Kulturschalen verwendet, bei denen die Zellen ungefähr eine Konfluenz von 50-60% aufwiesen. Zur Auswertung wurde jede Schale am übernächsten Tag vollständig am Fluoreszenzmikroskop bei 200-facher Vergrößerung gründlich auf Fluoreszenzsignale abgesucht. Da unbekannt war, wie stark die Expression des mtEGFP-Gens war, wurde die Zeitspanne bis zur Beobachtung absichtlich so groß gewählt.

Zu Beginn wurde das Plasmid pMAG12-1 verwendet, mit dem insgesamt 66 Kulturschalen mit der Zelllinie 143B.TK⁻ behandelt wurden. Allerdings konnten keine Zellen mit fluoreszierenden Mitochondrien gefunden werden. Mit der Fertigstellung des Plasmids pMAG13-1 wurden die Transfektionsversuche mit pMAG12-1 eingestellt. Der Vektor pMAG13-1 enthielt zusätzlich noch einen Replikationsursprung für den L-Strang, so dass eine Vermehrung innerhalb der Mitochondrien stattfinden konnte und somit eine Verstärkung der Expression möglich war. Das Plasmid wurde mit den gleichen Bedingungen wie sein Vorgänger pMAG12-1 für die Transfektionsversuche eingesetzt. Es kam wiederum die Zelllinie 143B.TK⁻ zum Einsatz, welche 96-mal behandelt wurde. Beim Absuchen der Platten konnten sehr schwache Fluoreszenzsignale entdeckt werden, die nach dem Vergleich mit dem entsprechenden Phasenkontrastbild eine Lokalisierung der Signale in den Mitochondrien der Zelle ergaben (Abb. 22). Zwei Tage später konnten die Zellen jedoch nicht mehr aufgefunden werden.

Um die Zellen bzw. ihre Mitochondrien einem Selektionsdruck für die eingebrachten Plasmide zu unterwerfen, wurde ein weiterer mitochondrialer Expressions-vektor eingesetzt: Bei dem Plasmid pMAG14-1 war zusätzlich zu den Genen des Plasmids pMAG13-1 noch ein 16S rRNA-Gen integriert, das eine Resistenz gegen das Antibiotikum Chloramphenicol vermittelt.

Nach der Behandlung von 72 Schalen der Zelllinie 143B.TK⁻ konnten keine fluoreszierenden Mitochondrien gefunden werden. Die gleichzeitige Zugabe von Chloramphenicol in das Kulturmedium erlaubte die Nutzung einer zusätzlichen Screeningmethode, die mit der Selektion von mit mtDNA transfizierten ρ⁰-Zellen übereinstimmte. Die Zellen wurden jeweils auf 150-mm-Schalen umgesetzt und nach zwei bis drei Wochen Kultivierung im Selektionsmedium mit Chloramphenicol auf Klone hin untersucht. Allerdings konnten auch mit dieser Methode keine Transfektanten nachgewiesen werden. Um die Expression des mtEGFP noch zu verstärken, wurden daraufhin Vektoren entwickelt, bei denen das mtEGFP-Gen durch die hochprozessive T7-RNA-Polymerase transkribiert werden sollte. In einem ersten Schritt wurde versucht, das Plasmid pCRII-TOPO mtEGFP (Abb. 9) mit der Genkanone in Zellen zu transfizieren, die die T7-RNA-Polymerase mit einer N-terminalen mitochondrialen Signalsequenz im Kern exprimierten. Bei einer geglückten Transfektion würde somit die in die Mitochondrien importierte T7-RNA-Polymerase das mtEGFP-Gen auf dem Plasmid transkribieren und ein Fluoreszenzsignal wäre sichtbar.

Das Plasmid wurde entsprechend der bisher verwendeten mitochondrialen Expressionsplasmide in 60 Transfektionsversuchen eingesetzt, jedoch konnten auch hier keine transfizierten Zellen am Fluoreszenzmikroskop gefunden werden.

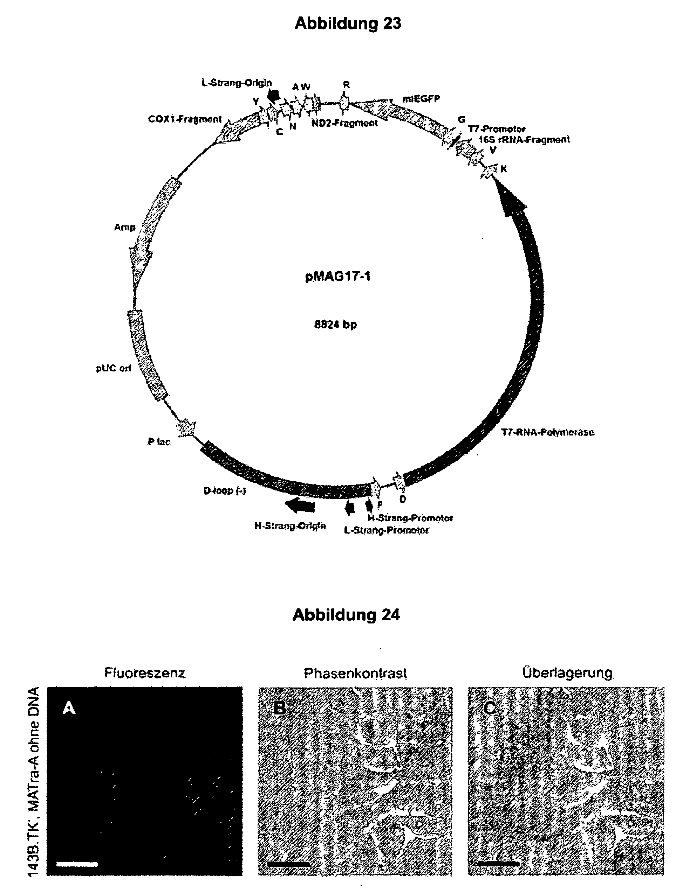

Anschließend wurden die Plasmide pMAG17-1 (Abb. 23) und pMAG14-3 bei den Transfektionsversuchen eingesetzt, die beide das Gen für die T7-RNA-Polymerase unter der Kontrolle des mitochondrialen H-Strang-Promotors enthielten. Bei diesen beiden Plasmiden zeigte sich - ähnlich wie bei den Transfektionsversuchen mit der mitochondrialen DNA - eine Aggregation der Goldpartikel, die aber nicht so stark war und durch die Reduktion der pro Versuch eingesetzten DNA-Menge vollständig verhindert werden konnte. Dementsprechend wurden nur 0,5 µg Plasmid-DNA auf 0,5 µg Goldpartikel eingesetzt. Nachdem 78 Schalen mit dem Plasmid pMAG17-1 (Abb. 23) behandelt wurden, konnten wiederum keine Transfektanten gefunden werden und es wurde mit dem Plasmid pMAG14-3 fortgefahren, das zusätzlich das 16S rRNA-Gen für die Chloramphenicol-Resistenz enthielt. Nach 66 Transfektionsversuchen wurden auch bei diesem Plasmid keine fluoreszierenden Mitochondrien am Fluoreszenzmikroskop entdeckt, und auch die Selektion auf die Chloramphenicol-Resistenz endete ohne positives Ergebnis.

### 13.2. Transfektion mit magnetischen Partikeln

Bei den im Rahmen der vorliegenden Erfindung durchgeführten Versuchen mit MATra-A, wurden die Megamitochondrien durch eine eintägige Inkubation in mit Milchsäure auf einen pH-Wert von 6,3 eingestelltem Kulturmedium (vgl. Beispiel 13.1.3) in 35-mm-Kulturschalen induziert. Anschließend erfolgte die eigentliche Transfektion mit 3 µg des entsprechenden Plasmids.

Nach der Transfektion mit dem Plasmid pMAG13-1 konnten nach zwei Tagen mit dem Fluoreszenzmikroskop überall auf der Schale leicht grün leuchtende Strukturen entdeckt werden. Da die, nach den Genkanone-Versuchen erwartete Intensität der Fluoreszenzsignale ebenfalls nur sehr niedrig war, wurden diese Strukturen näher untersucht. Aufgrund von Vergleichen mit den entsprechenden Phasenkontrastbildern wurden die Partikel als Auslöser der Fluoreszenz verdächtigt. Nach dem Einsatz von MATra-A-Partikeln ohne Plasmid-DNA konnten diese Strukturen wiederum in bzw. auf den Zellen entdeckt werden (Abb. 24), was diese Vermutung bestätigte.

Aufgrund dieser Beobachtungen wurden die mit MATra-A transfizierten Zellen nicht auf die Expression des mtEGFP-Gens hin untersucht. Daraufhin erfolgten noch 25 Transfektionen mit dem Plasmid pMAG14-1, das eine Chloramphenicol-Resistenz vermitteln kann. Nach dem Umsetzen der Zellen auf 150-mm-Kulturschalen konnten jedoch auch hier nach einer drei bis vier Wochen dauernden Weiterkultivierung keine Chloramphenicol-resistenten Klone gefunden werden.

### 13.3. Transfektion durch Mikroiniektion von DNA

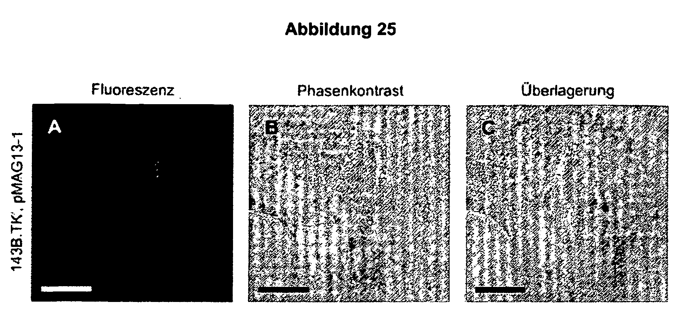

Die Zelllinie 143B.TK⁻ wurde auf Glasbodenschalen ausgesät und die Megamitochondrien mit Natriumacetat induziert (Beispiel 12.1). Das Plasmid pMAG13-1 fand in Konzentrationen von 50-350 ng/µl Verwendung, wobei die Injektionseinstellungen ebenfalls variiert wurden. Nach der Injektion wurden die Zellen mit Antibiotika weiterkultiviert, um mögliche Kontaminationen durch die Injektion an der offenen Schale zu minimieren. Am nächsten Tag wurden die Kulturschalen am Fluoreszenzmikroskop auf Transformanten abgesucht. Es konnten sehr schwache Signale innerhalb von Zellen entdeckt werden, die auf eine gelungene Transfektion von Mitochondrien schließen ließen (Abb.25). Injektionsversuche mit mitochondrialer DNA in ρ⁰-Zellen wurden nicht durchgeführt, da es in diesen Zellen nicht gelang, Megamitochondrien in entsprechender Größe zu induzieren.

## Patentansprüche

1. In-vitro Verfahren zum Einbringen einer zu exprimierenden DNA in Mitochondrien von Säugerzellen, wobei das Verfahren die Schritte umfasst:
(i)
- Konstruktion eines mitochondrialen Expressionsvektors umfassend (a) ein zu exprimierendes Gen und/oder einen Selektionsmarker und (b) einen mitochondrialen Promoterbereich, oder
- Konstruktion eines mitochondrialen Genoms,
(ii) reversible Induktion von Megamitochondrien und
(iii) Transfektion der Megamitochondrien mittels einer physikalischen Transfektionsmethode.

2. Verfahren nach Anspruch 1, wobei die Megamitochondrien durch Ansäuerung des Säugerzellen umfassenden Kulturmediums reversibel induziert werden.

3. Verfahren nach Anspruch 2, wobei das Kulturmedium durch Zugabe von Milchsäure, Lactat, Natriumacetat und/oder Essigsäure bzw. Gemischen davon angesäuert wird.

4. Verfahren nach Anspruch 3, wobei der pH-Wert im Kulturmedium zur Ansäuerung auf zwischen 5,7 und 7,0, vorzugsweise auf zwischen 6,3 und 7,0 eingestellt wird.

5. Verfahren nach Anspruch 3, wobei die Konzentration von Milchsäure, Natriumacetat und/oder Essigsäure im Kulturmedium nach der Ansäuerung bei zwischen 5 bis 100 mM liegt, vorzugsweise bei zwischen 40 bis 70 mM für Milchsäure, vorzugsweise bei zwischen 50 bis 60 mM für Natriumacetat und vorzugsweise bei zwischen 30 bis 35 mM für Essigsäure.

6. Verfahren nach einem der Ansprüche 1 bis 5, wobei die zu exprimierende DNA exogene DNA, vollständige mitochondriale DNA und/oder deren Derivate umfasst.

7. Verfahren nach einem der Ansprüche 1 bis 6, wobei die physikalische Transfektionsmethode aus der Gruppe ausgewählt ist, die aus dem Beschießen der Zellen mit DNA-beschichteten Mikropartikeln in einer Genkanone, der Transfektion mithilfe magnetischer Partikel und der Mikroinjektion besteht.

8. Verfahren nach Anspruch 7, wobei vor dem Beschießen der Säugerzellen mit DNA-beschichteten Mikropartikeln in einer Genkanone oder vor der Transfektion mithilfe magnetischer Partikel die Megamitochondrien durch Ansäuerung des Kulturmediums mittels Zugabe von Milchsäure reversibel induziert werden.

9. Verfahren nach Anspruch 7 oder 8, wobei vor der Mikroinjektion der Säugerzellen die Megamitochondrien durch Ansäuerung des Kulturmediums mittels Zugabe von Natriumacetat oder Essigsäure reversibel induziert werden.

10. Verfahren nach einem der Ansprüche 1 bis 9, wobei der mitochondriale Expressionsvektor einen mitochondrialen Promotorbereich und ein zu exprimierendes Gen umfasst oder aus dem kompletten mitochondrialen Genom besteht oder Derivate desselben umfasst.

11. In-vitro Verfahren zur Transfektion von Megamitochondrien von Säugerzellen mittels einer physikalischen Transfektionsmethode, wobei die physikalische Transfektionsmethode aus der Gruppe ausgewählt ist, die aus dem Beschießen der Zellen mit DNA-beschichteten Mikropartikeln in einer Genkanone, der Transfektion mithilfe magnetischer Partikel und der Mikroinjektion besteht.

12. Verfahren nach Anspruch 11, wobei vor dem Beschießen der Säugerzellen mit DNA-beschichteten Mikropartikeln in einer Genkanone oder vor der Transfektion mithilfe magnetischer Partikel die Megamitochondrien durch Ansäuerung des Kulturmediums mittels Zugabe von Milchsäure reversibel induziert werden.

## Claims

1. In vitro method for introducing a DNA to be expressed into mitochondria from mammalians, wherein the method comprises the steps of:
(i)
- design of a mitochondrial expression vector, comprising (a) gene to be expressed and/or a selection marker and (b) a mitochondrial promotor region, or
- design of a mitochondrial genome,
(ii) reversible induction of megamitochondria and
(iii) transfection of the megamitochondria by means of a physical transfection method.

2. The method according to claim 1, wherein the megamitochondria are reversibly induced by acidifying the culture medium comprising mammalian cells.

3. The method according to claim 2, wherein the culture medium is acidified by adding lactic acid, lactate, sodium acetate and/or acetic acid and/or mixtures thereof.

4. The method according to claim 3, wherein for the purpose of acidification the pH value in the culture medium is adjusted to between 5.7 and 7.0, preferably between 6.3 and 7.0.

5. The method according to claim 3, wherein the concentration of lactic acid, sodium acetate and/or acetic acid in the culture medium after the acidification is between 5 to 100 mM, preferably between 40 and 70 mM for lactic acid, preferably between 50 and 60 mM for sodium acetate and preferably between 30 and 35 mM for acetic acid.

6. The method according to any of claims 1 to 5, wherein the DNA to be expressed comprises exogenous DNA, complete mitochondrial DNA and/or the derivatives thereof.

7. The method according to any of claims 1 to 6, wherein the physical transfection method is selected from the group consisting of the bombardment of the cells with DNA-coated microparticles in a gene gun, the transfection by means of magnetic particles and the microinjection.

8. The method according to claim 7, wherein prior to the bombardment of the mammalian cells with DNA-coated microparticles in a gene gun or prior to the transfection by means of magnetic particles the megamitochondria are reversibly induced by acidification of the culture medium by adding lactic acid.

9. The method according to claim 7 or 8, wherein prior to the microinjection of the mammalian cells the megamitochondria are reversibly induced by the acidification of the culture medium by adding sodium acetate or acetic acid.

10. The method according to any of claims 1 to 9, wherein the mitochondrial expression vector comprises a mitochondrial promotor region and a gene to be expressed or consists of the complete mitochondrial genome or comprises derivatives thereof.

11. In vitro methods for the transfection of megamitochondria of mammalian cells by means of a physical transfection method, wherein the physical transfection method is selected from the group consisting of the bombardment of the cells by means of DNA-coated microparticles in a gene gun, the transfection by means of magnetic particles and the microinjection.

12. The method according to claim 11, wherein prior to the bombardment of the mammalian cells with DNA-coated microparticles in a gene gun or prior to the transfection by means of magnetic particles the megamitochondria are reversibly induced by acidification of the culture medium by adding lactic acid.

## Revendications

1. Procédé in-vitro pour introduire un ADN à exprimer dans des mitochondries de cellules de mammifère, le procédé comprenant les étapes de:
(i)
- construction d'un vecteur d'expression mitochondrial comportant (a) un gène à exprimer et/ou un marqueur de sélection et (b) une région de promoteur mitochondrial, ou
- construction d'un génome mitochondrial,
(ii) induction réversible de méga-mitochondries, et
(iii) transfection des méga-mitochondries au moyen d'une méthode de transfection physique.

2. Procédé selon la revendication 1, dans lequel les méga-mitochondries sont induites de manière réversible par acidification du milieu de culture comportant des cellules de mammifère.

3. Procédé selon la revendication 2, dans lequel le milieu de culture est acidifié par addition d'acide lactique, de lactate, d'acétate de sodium et/ou d'acide acétique ou de mélanges de ceux-ci.

4. Procédé selon la revendication 3, dans lequel la valeur de pH dans le milieu de culture est, pour l'acidification, réglée entre 5,7 et 7,0, de préférence entre 6,3 et 7,0.

5. Procédé selon la revendication 3, dans lequel la concentration de l'acide lactique, de l'acétate de sodium et/ou de l'acide acétique dans le milieu de culture se situe, après l'acidification, entre 5 et 100 mM, de préférence entre 40 et 70 mM pour l'acide lactique, de préférence entre 50 à 60 mM pour l'acétate de sodium, et de préférence entre 30 et 35 mM pour l'acide acétique.

6. Procédé selon l'une des revendications 1 à 5, dans lequel l'ADN à exprimer comprend de l'ADN exogène, de l'ADN entièrement mitochondrial et/ou leurs dérivés.

7. Procédé selon l'une des revendications 1 à 6, dans lequel la méthode de transfection physique est choisie parmi le groupe composé du bombardement des cellules par des microparticules revêtues d'ADN dans un canon à gènes, de la transfection à l'aide de particules magnétiques, et de la micro-injection.

8. Procédé selon la revendication 7, dans lequel, avant le bombardement des cellules de mammifères par des microparticules revêtues d'ADN dans un canon à gènes ou avant la transfection à l'aide de particules magnétiques, les mégamitochondries sont induites de manière réversible par acidification du milieu de culture par addition d'acide lactique.

9. Procédé selon la revendication 7 ou 8, dans lequel, avant la micro-injection des cellules de mammifères, les méga-mitochondries sont induites de manière réversible par acidification du milieu de culture par addition d'acétate de sodium ou d'acide acétique.

10. Procédé selon l'une des revendications 1 à 9, dans lequel le vecteur d'expression mitochondrial comprend une région de promoteur mitochondrial et un gène à exprimer, ou est constitué du génome mitochondrial complet ou comprend des dérivés de ce dernier.

11. Procédé in vitro pour la transfection de méga-mitochondries de mammifères à l'aide d'une méthode de transfection physique, dans lequel la méthode de transfection physique est choisie parmi le groupe composé du bombardement des cellules par des microparticules revêtues d'ADN dans un canon à gènes, de la transfection à l'aide de particules magnétiques, et de la micro-injection.

12. Procédé selon la revendication 11, dans lequel, avant le bombardement des cellules de mammifères par des microparticules revêtues d'ADN dans un canon à gènes ou avant la transfection à l'aide de particules magnétiques, les mégamitochondries sont induites de manière réversible par acidification du milieu de culture par addition d'acide lactique.
